# EUROPEAN PATENT SPECIFICATION

(11) **EP 1 663 398 B1**
(45) Date of publication and mention of the grant of the patent: **25.11.2009**
(21) Application number: 04769341.1
(22) Date of filing: 06.09.2004
(51) Int. Cl.: A61P 29/00, A61P 25/02, A61P 25/04, A61P 25/06, A61K 45/06, A61K 31/196, A61K 31/197, A61K 31/136, A61K 31/5375, A61K 31/381, A61K 31/165, A61K 31/137

(54) **COMBINATIONS COMPRISING ALPHA-2-DELTA LIGANDS AND SEROTONIN / NORADRENALINE REUPTAKE INHIBITORS**
KOMBINATIONEN AUS ALPHA-2-DELTA LIGANDEN UND SEROTONIN / NORADRENALIN-WIEDERAUFNAHMEHEMMERN
ASSOCIATIONS COMPRENANT DES LIGANDS ALPHA-2-DELTA ET DES INHIBITEURS DU RECAPTAGE DE LA SEROTONINE ET DE LA NORADRENALINE

(30) Priority: 12.09.2003 US 502556 P
(43) Date of publication of application: 07.06.2006
(73) Proprietor: Pfizer Limited, Sandwich, Kent CT13 9NJ (GB); Pfizer Inc., New York, NY 10017 (US)
(72) Inventor: DOOLEY, David J., Ann Arbor, MI 48105 (US); FIELD, Mark John, Sandwich, Kent CT13 9NJ (GB); WILLIAMS, Richard Griffith, Sandwich, Kent CT13 9 NJ (GB)
(74) Representative: Summers, Victoria Clare
(86) International application number: PCT/IB2004/002943
(87) International publication number: WO 2005/025675

(56) References cited:
- EP-A- 1 254 660
- WO-A-00/61234
- WO-A-99/09980
- WO-A-03/061656
- WO-A-03/070237
- WO-A-2004/054560
- US-B1- 6 372 792
- WALDMAN S D ET AL: "THE PHARMACOLOGIC MANAGEMENT OF NEUROPATHIC PAIN" PROGRESS IN ANESTHESIOLOGY, DANNEMILLER MEMORIAL EDUCATIONAL FOUNDATION, SAN ANTONIO, TX,, US, vol. 12, no. 4, 1998, pages 55-64, XP000982774 ISSN: 0891-5784
- VILLANI A ET AL: "Phantom art pain prevention with Gabapentin and Mirtazapine combination: Case report" IMPEGNO OSPEDALIERO, SEZIONE SCIENTIFICA 2003 ITALY, vol. 24, no. 2, 2003, pages 53-54, XP008040562 ISSN: 0393-0394
- PRIDMORE S ET AL: "Will the atypical antipsychotics be analgesics?" AUSTRALASIAN PSYCHIATRY 2003 AUSTRALIA, vol. 11, no. 1, March 2003 (2003-03), pages 59-61, XP008040570 ISSN: 1039-8562
- KENNEDY S H ET AL: "Reboxetine: A preliminary report on its use through the Special Access Program" JOURNAL OF PSYCHIATRY AND NEUROSCIENCE 2002 CANADA, vol. 27, no. 6, 2002, pages 418-422, XP008040561 ISSN: 1180-4882
- RAO S G ET AL: "PHARMACOLOGICAL THERAPIES IN FIBROMYALGIA" BAILLIERE'S BEST PRACTICE AND RESEARCH. CLINICAL REUMATOLOGY, BAILLIERE TINDALL, LONDON, GB, vol. 17, no. 4, 2003, pages 611-627, XP009026987 ISSN: 1521-6942
- GUAY D R P: "Adjunctive agents in the management of chronic pain" PHARMACOTHERAPY 2001 UNITED STATES, vol. 21, no. 9 I, 2001, pages 1070-1081, XP008040573 ISSN: 0277-0008

## Description

### FIELD OF THE INVENTION

This invention relates to a synergistic combination of an alpha-2-delta ligand selected from gabapentin and pregabalin and a selective noradrenaline re-uptake inhibitor (SNRI), viz. (S,S)-reboxetine, for the treatment of pain. It also relates to treating pain through the use of effective amounts of synergistic combinations of an alpha-2-delta ligand and SNRI, as described above.

### BACKGROUND TO THE INVENTION

An alpha-2-delta receptor ligand is any molecule which binds to any sub-type of the human calcium channel alpha-2-delta sub-unit. The calcium channel alpha-2-delta sub-unit comprises a number of receptor sub-types which have been described in the literature: e.g. N. S. Gee, J. P. Brown, V. U. Dissanayake, J. Offord, R. Thurlow, and G. N. Woodruff, J-Biol-Chem 271 (10):5768-76, 1996, (type 1); Gong, J. Hang, W. Kohler, Z. Li, and T-Z. Su, J.Membr.Biol. 184 (1):35-43, 2001, (types 2 and 3); E. Marais, N. Klugbauer, and F. Hofmann, Mol.Pharmacol. 59 (5):1243-1248, 2001. (types 2 and 3); and N. Qin, S. Yagel, M. L. Momplaisir, E. E. Codd, and M. R. D'Andrea. Mol.Pharmacol. 62 (3):485-496, 2002, (type 4). They may also be known as GABA analogs.

Alpha-2-delta ligands have been described for a number of indications. The best known alpha-2-delta ligand, gabapentin (Neurontin®), 1-(aminomethyl)-cyclohexylacetic acid, was first described in the patent literature in the patent family comprising US4024175. The compound is approved for the treatment of epilepsy and neuropathic pain.

A second alpha-2-delta ligand, pregabalin, (S)-(+)-4-amino-3-(2-methylpropyl)butanoic acid, is described in European patent application publication number EP641330 as an anti-convulsant treatment useful in the treatment of epilepsy and in EP0934061 for the treatment of pain.

Further, International Patent Application Publication No. WO0128978, describes a series of novel bicyclic amino acids, their pharmaceutically acceptable salts, and their prodrugs of formula: 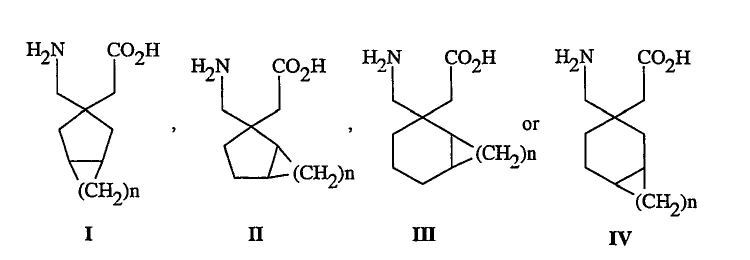
wherein n is an integer of from 1 to 4, where there are stereocentres, each center may be independently R or S, preferred compounds being those of Formulae I-IV above in which n is an integer of from 2 to 4.

More recently, International Patent Application Publication Number WO02/85839 describes alpha-2-delta ligands of the following formulae: 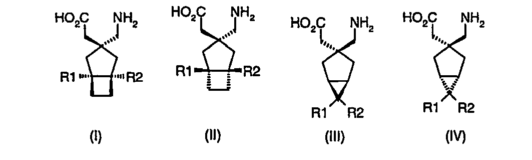 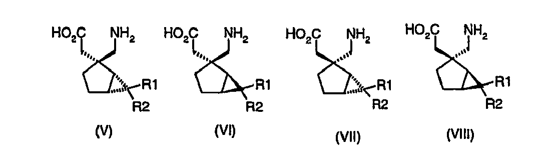 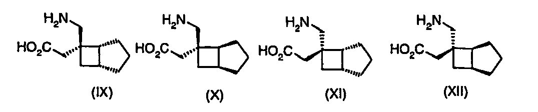 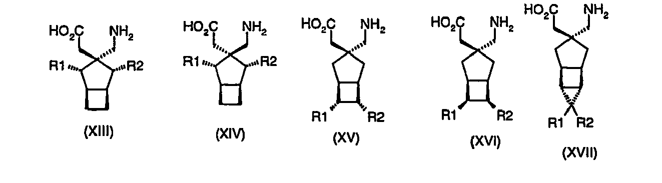 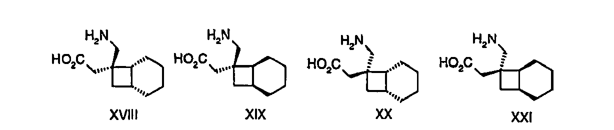 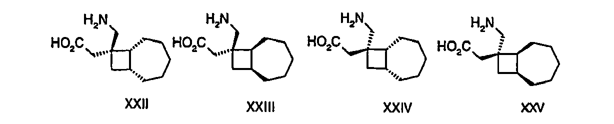
wherein R¹ and R² are each independently selected from H, straight or branched alkyl of 1-6 carbon atoms, cycloalkyl of from 3-6 carbon atoms, phenyl and benzyl, subject to the proviso that, except in the case of a tricyclooctane compound of formula (XVII), R¹ and R² are not simultaneously hydrogen; for use in the treatment of a number of indications, including pain, together with combinations with: selective serotonin reuptake inhibitors, e.g. fluoxetine, paroxetine, citalopram and sertraline; mixed serotonin-noradrenaline reuptake inhibitors, e.g. milnacipran, venlafaxine and duloxetine; and selective noradrenaline reuptake inhibitors , e.g. reboxetine.

International Patent application No. PCT/IB03/00976, unpublished at the filing date of the present invention, describes compounds of the formula I, below: 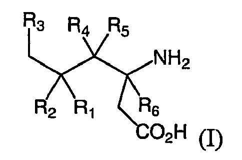
wherein R₁ is hydrogen or (C₁-C₆)alkyl optionally substituted with from one to five fluorine atoms;
R₂ is hydrogen or (C₁-C₆)alkyl optionally substituted with from one to five fluorine atoms; or
R₁ and R₂, together with the carbon to which they are attached, form a three to six membered cycloalkyl ring;
R₃ is (C₁-C₆)alkyl, (C₃-C₆)cycloalkyl, (C₃-C₆)cycloalkyl-(C₁-C₃)alkyl, phenyl, phenyl-(C₁-C₃)alkyl, pyridyl, pyridyl-(C₁-C₃)alkyl, phenyl-N(H)-, or pyridyl-N(H)- , wherein each of the foregoing alkyl moieties can be optionally substituted with from one to five fluorine atoms, preferably with from zero to three fluorine atoms, and wherein said phenyl and said pyridyl and the phenyl and pyridyl moieties of said phenyl-(C₁-C₃)alkyl and said pyridyl-(C₁-C₃)alkyl, respectively, can be optionally substituted with from one to three substituents, preferably with from zero to two substituents, independently selected from chloro, fluoro, amino, nitro, cyano, (C₁-C₃)alkylamino, (C₁-C₃)alkyl optionally substituted with from one to three fluorine atoms and (C₁-C₃)alkoxy optionally substituted with from one to three fluorine atoms;
R₄ is hydrogen or (C₁-C₆)alkyl optionally substituted with from one to five fluorine atoms;
R₅ is hydrogen or (C₁-C₆)alkyl optionally substituted with from one to five fluorine atoms; and
R₆ is hydrogen or (C₁-C₆)alkyl;
or a pharmaceutically acceptable salts thereof.

Many types of neurological disorders originate from disturbances in brain circuits that convey signals using certain monoamine neurotransmitters. Monoamine neurotransmitters include, for example, serotonin (5-HT), norepinephrine (noradrenaline), and dopamine- These neurotransmitters travel from the terminal of a neuron across a small gap (i.e., the synaptic cleft) and bind to receptor molecules on the surface of a second neuron. This binding elicits intracellular changes that initiate or activate a response or change in the postsynaptic neuron. Inactivation occurs primarily by transport (i.e., reuptake) of the neurotransmitter back into the presynaptic neuron.

Selective serotonin reuptake inhibitors (SSRIs) function by inhibiting the reuptake of serotonin by afferent neurons. SSRI's well known in the art include, but are not limited to sertraline (Zoloft^{®}), sertraline metabolite demethylsertraline, fluoxetine (Prozac^{®}), norfluoxetine (fluoxetine desmethyl metabolite), fluvoxamine (Luvox^{®}), paroxetine (Seroxat^{®}, Paxil^{®}) and its alternative formulation, Paxil-CR^{®}, citalopram (Celexa^{®}), citalopram metabolite desmethylcitalopram, escitalopram (Lexapro^{®}), d,l-fenfluramine (Pondimin®), femoxetine, ifoxetine, cyanodothiepin, litoxetine, dapoxetine, nefazodone (Serxone®), cericlamine and trazodone (Desyrel®).

Selective noradrenaline (or norepinephrine) uptake inhibitors (SNRIs) function by increasing noradrenaline levels. SNRI's well known in the art include, but are not limited to, reboxetine (Edronax^{®}) and all enantiomers of reboxetine, ie., (R/R,S/S,R/S,S/R), desipramine (Norpramin^{®}), maprotiline (Ludiomil^{®}), lofepramine (Gamanil^{®}), mirtazepine (Remeron^{®}), oxaprotiline, fezolamine, tomoxetine, mianserin (Bolvidon®), buproprion (Wellbutrin^{®}), buproprion metabolite hydroxybuproprion, nomifensine (Merital®) and viloxazine (Vivalan®).

Dual serotonin-noradrenaline re-uptake inhibitors (DSNRIs), which inhibit the reuptake of both serotonin and norepinephrine include venlafaxine (Effexor®), venlafaxine metabolite O-desmethylvenlafaxine, clomipramine (Anafranil®), clomipramine metabolite desmethylclomipramine, duloxetine (Cymbalta®), milnacipran and imipramine (Tofranil® or Janimine®).

### SUMMARY OF THE INVENTION

It has now been found that combination therapy with an alpha-2-delta ligand selected from gabapentin and pregabalin and a selective noradrenaline re-uptake inhibitor (SNRI), viz. (S,S)-reboxetine, results in improvement in the treatment of pain. Furthermore, when administered simultaneously, sequentially or separately, the alpha-2-delta ligand and the SNRI may interact in a synergistic manner to control pain. This synergy allows a reduction in the dose required of each compound, leading to a reduction in the side effects and enhancement of the clinical utility of the compounds.

In the following, "alpha-2-delta ligand" will refer to either gabapentin or pregabalin, while "SNRI" will refer to (S,S)-reboxetine.

Accordingly, the invention provides, as a first aspect, a combination product comprising an alpha-2-delta ligand and a selective noradrenaline re-uptake inhibitor (SNRI), or pharmaceutically acceptable salts thereof, with the proviso that the compounds (i)-(xxv) of WO02/85839 in combination with a serotonin reuptake inhibitor, particularly fluoxetine, paroxetine, citalopram and sertraline, a mixed serotonin-noradrenaline reuptake inhibitor, paticularly milnacipran, venlafaxine and duloxetine, and a noradrenaline reuptake inhibitor, particularly reboxetine are excluded.

As an alternative or further aspect, the invention provides a synergistic combination product comprising an alpha-2-delta ligand and SNRI, or pharmaceutically acceptable salts thereof.

Cyclic alpha-2-delta ligands are illustrated by the following formula (I): 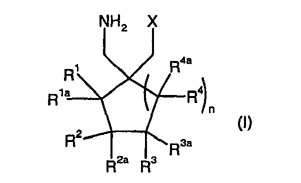
wherein X is a carboxylic acid or carboxylic acid bioisostere;
n is 0, 1 or 2; and
R¹, R^{1a}, R², R^{2a}, R³, R^{3a}, R⁴ and R^{4a} are independently selected from H and C₁-C₆ alkyl, or R¹ and R² or R² and R³ are taken together to form a C₃-C₇ cycloalkyl ring, which is optionally substituted with one or two substituents selected from C₁-C₆ alkyl, or a pharmaceutically acceptable salt thereof.

In formula (I), suitably, R¹, R^{1a}, R^{2a}, R^{3a}, R⁴ and R^{4a} are H and R² and R³ are independently selected from H and methyl, or R^{1a}, R^{2a}, R^{3a} and R^{4a} are H and R¹ and R² or R² and R³ are taken together to form a C₃-C₇ cycloalkyl ring, which is optionally substituted with one or two methyl substituents. A suitable carboxylic acid bioisostere is selected from tetrazolyl and oxadiazolonyl. X is preferably a carboxylic acid.

In formula (I), preferably, R¹, R^{1a}, R^{2a}, R^{3a}, R⁴ and R^{4a} are H and R² and R³ are independently selected from H and methyl, or R^{1a}, R^{2a}, R^{3a} and R^{4a} are H and R¹ and R² or R² and R³ are taken together to form a C₄-C₅ cycloalkyl ring, or, when n is 0, R¹, R^{1a}, R^{2a}, R^{3a}, R⁴ and R^{4a} are H and R² and R³ form a cyclopentyl ring, or, when n is 1, R¹, R^{1a}, R^{2a}, R^{3a}, R⁴ and R^{4a} are H and R² and R³ are both methyl or R¹, R^{1a}, R^{2a}, R^{3a}, R⁴ and R^{4a} are H and R² and R³ form a cyclobutyl ring, or, when n is 2, R¹, R^{1a}, R², R^{2a}, R³, R^{3a}, R⁴ and R^{4a} are H, or, n is 0, R¹, R^{1a}, R^{2a}, R^{3a}, R⁴ and R^{4a} are H and R² and R³ form a cyclopentyl ring.

Acyclic alpha-2-delta ligands are illustrated by the following formula (II): 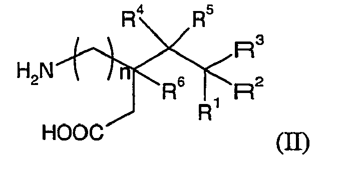 wherein:
n is 0 or 1, R¹ is hydrogen or (C₁-C₆)alkyl; R² is hydrogen or (C₁-C₆)alkyl; R³ is hydrogen or (C₁-C₆)alkyl; R⁴ is hydrogen or (C₁-C₆)alkyl; R⁵ is hydrogen or (C₁-C₆)alkyl and R² is hydrogen or (C₁-C₆)alkyl, or a pharmaceutically acceptable salt thereof.

According to formula (II), suitably R¹ is C₁-C₆ alkyl, R² is methyl, R³ - R⁶ are hydrogen and n is 0 or 1. More suitably R¹ is methyl, ethyl, n-propyl or n-butyl, R² is methyl, R³ - R⁶ are hydrogen and n is 0 or 1. When R² is methyl, R³ - R⁶ are hydrogen and n is 0, R¹ is suitably ethyl, n-propyl or n-butyl. When R² is methyl, R³ - R⁶ are hydrogen and n is 1, R¹ is suitably methyl or n-propyl. Compounds of formula (II) are suitably in the 3S,5R configuration.

Examples of alpha-2-delta ligands are those compounds generally or specifically disclosed in US4024175, particularly gabapentin, EP641330, particularly pregabalin, US5563175, WO9733858, WO9733859, WO9931057, WO9931074, WO9729101, WO02085839, particularly [(1R,5R,6S)-6-(Aminomethyl)bicyclo[3.2.0]hept-6-yl]acetic acid, WO993 1075, particularly 3-(1-Aminomethyl-cyclohexylmethyl)-4H-[1,2,4]oxadiazol-5-one and C-[1-(1H-Tetrazol-5-ylmethyl)-cycloheptyl]-methylamine, WO9921824, particularly (3S,4S)-(1-Aminomethyl-3,4-dimethyl-cyclopentyl)-acetic acid, WO0190052, WO0128978, particularly (1α,3α,5α)(3-amino-methyl-bicyclo[3.2.0]hept-3-yl)-acetic acid, EP0641330, WO9817627, WO0076958, particularly (3S,5R)-3-aminomethyl-5-methyl-octanoic acid, PCT/IB03/00976, particularly (3S,5R)-3-amino-5-methyl-heptanoic acid, (3S,5R)-3-amino-5-methyl-nonanoic acid and (3S,5R)-3-Amino-5-methyl-octanoic acid, EP1178034, EP1201240, WO9931074, WO03000642, WO0222568, WO0230871, WO0230881 WO02100392, WO02100347, WO0242414, WO0232736 and WO0228881 or pharmaceutically acceptable salts thereof.

Alpha-2-delta ligands include: gabapentin, pregabalin, [(1R,5R,6S)-6-(Aminomethyl)bicyclo[3.2.0]hept-6-yl]acetic acid, 3-(1-Aminomethyl-cyclohexylmethyl)-4H-[1,2,4]oxadiazol-5-one, C-[1-(1H-Tetrazol-5-ylmethyl)-cycloheptyl]-methylamine, (3S,4S)-(1-Aminomethyl-3,4-dimethyl-cyclopentyl)-acetic acid, (1α,3α,5α)(3-amino-methyl-bicyclo[3.2.0]hept-3-yl)-acetic acid, (3S,5R)-3-Aminomethyl-5-methyl-octanoic acid, (3S,5R)-3-amino-5-methyl-heptanoic acid, (3S,5R)-3-amino-5-methyl-nonanoic acid and (3S,5R)-3-Amino-5-methyl-octanoic acid, or pharmaceutically acceptable salts thereof.

SSRIs include those comprised within the disclosure of US4536518, i.e. the cis-isomeric compounds of formula (III): 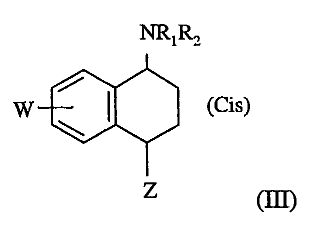
wherein R₁ is selected from the group consisting of hydrogen and normal alkyl of from 1 to 3 carbon atoms, R₂ is normal alkyl of from 1 to 3 carbon atoms, Z is 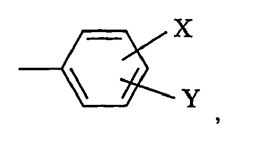
X and Y are each selected from the group consisting of hydrogen, fluoro, chloro, bromo, trifluoromethyl, alkoxy of from 1 to 3 carbon atoms and cyano, with at least one of X and Y being other than hydrogen, and W is selected from the group consisting of hydrogen, fluoro, chloro, bromo, trifluoromethyl and alkoxy of from 1 to 3 carbon atoms and wherein the term "cis-isomeric" refers to the relative orientation of the NR₁R₂ and Z moieties on the cyclohexene ring with said compound being either the (1S)-enantiomer or the racemic mixture of the (1S)-enantiomer with the corresponding (1R)-enantiomer or a prodrug thereof or a pharmaceutically acceptable salt thereof or of said prodrug. A compound of formula (III) is sertraline.

Examples of SSRIs are the compounds generically and specifically disclosed in U.S. 4,536,518, particularly sertraline, U.S. 4,943,590 [RE 34,712], U.S. 4,650,884, particularly citalopram, U.S. 3,198,834, particularly d,l-fenfluramine, U.S. 3,912,743, 4,571,424, particularly femoxetine, U.S. 4,314,081, 4,626,549 particularly fluoxetine, U.S. 4,085,225, particularly fluvoxetine, U.S. 3,912,743, 4,007,196, particularly paroxetine, ifoxetine, cyanodothiepin and litoxetine, or pharmaceutically acceptable salts thereof.

SSRIs include sertraline, sertraline metabolite demethylsertraline, fluoxetine, norfluoxetine (fluoxetine desmethyl metabolite), fluvoxamine, paroxetine and its alternative formulation, Paxil-CR^{®}, citalopram, citalopram metabolite desmethylcitalopram, escitalopram, d,l-fenfluramine, femoxetine, ifoxetine, cyanodothiepin, litoxetine, dapoxetine, nefazaodone, cericlamine and trazodone, or pharmaceutically acceptable salts thereof.

SNRI's include the compounds disclosed in US4229449, i.e. the racemates and optical isomers corresponding to a compound of the formula (IV) 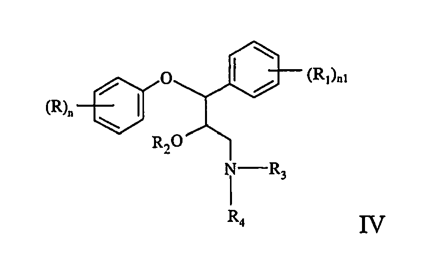 preferably the substituted propanolamine and morpholine derivatives, corresponding to formula IV, wherein
n and n₁ are, independently, 1, 2 or 3;
each of the groups R and R₁, which may be the same or different, is hydrogen; halogen; halo-C₁ -C₆ alkyl; hydroxy; C₁ -C₆ alkoxy; C₁ -C₆ alkyl optionally substituted; aryl- C₁ -C₆ alkyl optionally substituted; aryl- C₁ -C₆ alkoxy optionally substituted; -NO₂ ; 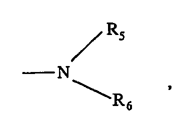
wherein R₅ and R₆ are, independently, hydrogen or C₁ -C₆ alkyl, or two adjacent R groups or two adjacent R₁ groups, taken together, form the -O-CH₂-O- radical;
R₂ is hydrogen; C₁ -C₁₂ alkyl optionally substituted, or aryl- C₁ -C₆ alkyl;
each of the groups R₃ and R₄, which may be identical or different, is hydrogen, C₁ -C₆ alkyl optionally substituted, C₂ -C₄ alkenyl, C₂ -C₄ alkynyl, aryl- C₂ -C₄ alkyl optionally substituted, C₃ -C₇ cycloalkyl optionally substituted, or R₃ and R₄ with the nitrogen atom to which they are bonded form a pentatomic or hexatomic saturated or unsaturated, optionally substituted, heteromonocyclic radical optionally containing other heteroatoms belonging to the class of O,S and N; or R₂ and R₄, taken together, form the -CH.₂ -CH₂- radical. A preferred compound of formula (IV) is represented by reboxetine.

Examples of SNRIs are the compounds generically and specifically disclosed in U.S. 4,229,449, 5,068,433, 5,391,735, particularly reboxetine, BP 908,788, 980,231, U.S. 3,454,554, particularly desipramine, U.S. 3,399,201, particularly maprotiline, BP 1,177,525, U.S. 3,637,660, particularly lofepramine, Neth. Pat. Appl. 6,603,256, U.S. 3,534,041, particularly mianserin, U.S. 4,062,843, particularly mirtazepine; U.S. 4,314,081, 4,018,895, 4,194,009, particularly tomoxetine, U.S. 4,535,186, 4,611,078, particularly venlafaxine, and U.S. 3,819,706, 3,885,046, particularly buproprion, and oxaprotiline and fezolamine, or pharmaceutically acceptable salts thereof.

Specific examples of SNRIs include reboxetine and all enantiomers of reboxetine, ie., (R/R,S/S,R/S,S/R), desipramine , maprotiline, lofepramine, mirtazepine, venlafaxine (described in US Patent No.4,761,501), oxaprotiline, fezolamine, tomoxetine, mianserin and buproprion, buproprion metabolite hydroxybuproprion, nomifensine or viloxazine, or a pharmaceutically acceptable salt thereof.

DSNRIs may be illustrated by the compounds of formula (V) 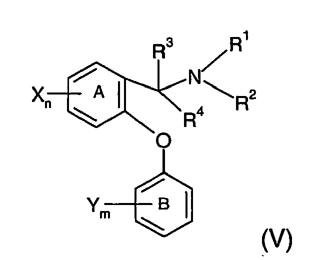
wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
each X is selected, independently, from hydrogen, halo (i.e., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Y is selected, independently, from hydrogen, (C₁-C₆)alkyl and halo;
with the proviso that: (a) no more than one of NR¹R², CR³R⁴ and R²NCR³ can form a ring; and (b) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups; and the pharmaceutically acceptable salts thereof. Compounds according to formula V are described in WO 00/50380.

DSNRIs are venlafaxine, venlafaxine metabolite O-desmethylvenlafaxine, clomipramine, clomipramine metabolite desmethylclomipramine, duloxetine, milnacipran and imipramine, or a pharmaceutically acceptable salt thereof.

Particular DSNRIs, SSRIs or SNRIs can be readily determined by evaluation of potency and selectivity using literature methods followed by evaluation of its toxicity, absorption, metabolism, pharmacokinetics, etc in accordance with standard pharmaceutical practices.

As an aspect of the present invention, there is provided a combination comprising gabapentin, or a pharmaceutically acceptable salt thereof and an SNRI selected from S,S-reboxetine or a pharmaceutically acceptable salt thereof, and their pharmaceutically acceptable salts.

As an aspect of the present invention, there is provided a combination comprising pregabalin and an SNRI selected from S,S-reboxetine or a pharmaceutically acceptable salt thereof, and their pharmaceutically acceptable salts.

As an aspect of the present invention, the combination is selected from:
gabapentin and S,S-reboxetine;
pregabalin and S,S-reboxetine
or pharmaceutically acceptable salts thereof.

The combination of the present invention in a single dosage form is suitable for administration to any mammalian subject, preferably human. Administration may be once (o.d.), twice (b.i.d.) or three times (t.i.d.) daily, suitably b.i.d. or t.i.d., more suitably b.i.d, most suitably o.d..

Thus, as a further aspect of the present invention, there is provided the use of a combination, particularly synergistic, of an alpha-2-delta ligand and SNRI in the manufacture of a once, twice or thrice, suitably twice or thrice, more suitably twice, most suitably once daily administration medicament for the curative, prophylactic or palliative treatment of pain.

Alternatively, the re is provided a method for the curative, prophylactic or palliative treatment of pain in a mammalian subject comprising once, twice or thrice, suitably twice or thrice, more suitably twice, most suitably once daily administration of an effective, particularly synergistic, combination of an alpha-2-delta ligand and SNRI.

Determining a synergistic interaction between one or more components, the optimum range for the effect and absolute dose ranges of each component for the effect may be definitively measured by administration of the components over different w/w ratio ranges and doses to patients in need of treatment. For humans, the complexity and cost of carrying out clinical studies on patients renders impractical the use of this form of testing as a primary model for synergy. However, the observation of synergy in one species can be predictive of the effect in other species and animal models exist, as described herein, to measure a synergistic effect and the results of such studies can also be used to predict effective dose and plasma concentration ratio ranges and the absolute doses and plasma concentrations required in other species by the application of pharmacokinetic/pharmacodynamic methods. Established correlations between animal models and effects seen in man suggest that synergy in animals is best-demonstrated using static and dynamic allodynia measurements in rodents that have undergone surgical (e.g. chronic constriction injury) or chemical (e.g. streptozocin) procedures to induce the allodynia. Because of plateau effects in such models, their value is best assessed in terms of synergistic actions that in neuropathic pain patients would translate to dose-sparing advantages. Other models in which existing agents used for the treatment of neuropathic pain give only a partial response are more suited to predict the potential of combinations acting synergistically to produce increased maximal efficacy at maximally tolerated doses of the two components.

Thus, as a further aspect of the present invention, there is provided a synergistic combination for human administration comprising an alpha-2-delta ligand and an SNRI, or pharmaceutically acceptable salts thereof, in a w/w combination range which corresponds to the absolute ranges observed in a non-human animal model, preferably a rat model, primarily used to identify a synergistic interaction. Suitably, the ratio range in humans corresponds to a non-human range selected from between 1:50 to 50:1 parts by weight, 1:50 to 20:1, 1:50 to 10:1, 1:50 to 1:1, 1:20 to 50:1, 1:20 to 20:1, 1:20 to 10:1, 1:20 to 1:1, 1:10 to 50:1, 1: 10 to 20:1, 1:10 to 10:1, 1:10 to 1:1, 1:1 to 50:1, 1.1 to 20:1 and 1:1 to 10:1. More suitably, the human range corresponds to a non-human range of 1:10 to 20:1 parts by weight. Preferably, the human range corresponds to a synergistic non-human range of the order of 1:1 to 10:1 parts by weight.

For humans, several experimental pain models may be used in man to demonstrate that agents with proven synergy in animals also have effects in man compatible with that synergy. Examples of human models that may be fit for this purpose include the heat/capsaicin model (Petersen, K.L. & Rowbotham, M.C. (1999) NeuroReport 10, 1511-1516), the i.d capsaicin model (Andersen, O.L., Felsby, S., Nicolaisen, L., Bjerring, P., Jsesn, T.S. & Arendt-Nielsen, L. (1996) Pain 66, 51-62), including the use of repeated capsaicin trauma (Witting, N., Svesson, P., Arendt-Nielsen, L. &Jensen, T.S. (2000) Somatosensory Motor Res. 17, 5-12), and summation or wind-up responses (Curatolo, M. et al. (2000) Anesthesiology 93, 1517 - 1530). With these models, subjective assessment of pain intensity or areas of hyperalgesia may be used as endpoints, or more objective endpoints, reliant on electrophysiological or imaging technologies (such as functional magnetic resonance imaging) may be employed (Bornhovd, K., Quante, M., Glauche, V., Bromm, B., Weiller, C. & Buchel, C. (2002) Brain 125, 1326-1336). All such models require evidence of objective validation before it can be concluded that they provide evidence in man of supporting the synergistic actions of a combination that have been observed in animal studies.

For the present invention in humans, a suitable alpha-2-delta ligand: SNRI ratio range is selected from between 1:50 to 50:1 parts by weight, 1:50 to 20:1, 1:50 to 10:1, 1:50 to 1:1, 1:20 to 50:1, 1:20 to 20:1, 1:20 to 10:1, 1:20 to 1:1, 1:10 to 50:1, 1:10 to 20:1, 1:10 to 10:1, 1:10 to 1:1, 1:1 to 50:1, 1.1 to 20:1 and 1:1 to 10:1, more suitably 1:10 to 20:1, preferably, 1:1 to 10:1.

Optimal doses of each component for synergy can be determined according to published procedures in animal models. However, in man (even in experimental models of pain) the cost can be very high for studies to determine the entire exposure-response relationship at all therapeutically relevant doses of each component of a combination. It may be necessary, at least initially, to estimate whether effects can be observed that are consistent with synergy at doses that have been extrapolated from those that give optimal synergy in animals. In scaling the doses from animals to man, factors such as relative body weight/body surface area, relative absorption, distribution, metabolism and excretion of each component and relative plasma protein binding need to be considered and, for these reasons, the optimal dose ratio predicted for man (and also for patients) is unlikely to be the same as the dose ratio shown to be optimal in animals. However, the relationship between the two can be understood and calculated by one skilled in the art of animal and human pharmacokinetics. Important in establishing the bridge between animal and human effects are the plasma concentrations obtained for each component used in the animal studies, as these are related to the plasma concentration of each component that would be expected to provide efficacy in man. Pharmacokinetic/ pharmacodynamic modeling (including methods such as isobolograms, interaction index and response surface modelling) and simulations may help to predict synergistic dose ratios in man, particularly where either or both of these components has already been studied in man.

It is important to ascertain whether any concluded synergy observed in animals or man is due solely to pharmacokinetic interactions. For example, inhibition of the metabolism of one compound by another might give a false impression of pharmacodynamic synergy.

Thus, according to a further aspect of the present invention, there is provided a synergistic combination for administration to humans comprising an alpha-2-delta ligand and SNRI or pharmaceutically acceptable salts thereof, where the dose range of each component corresponds to the absolute ranges observed in a non-human animal model, preferably the rat model, primarily used to identify a synergistic interaction.

Suitably, the dose of alpha-2-delta ligand for use in a human is in a range selected from 1-1200mg, 1-500mg, 1-100mg, 1-50mg, 1-25mg, 500-1200mg, 100-1200mg, 100-500mg, 50-1200mg, 50-500mg, or 50-100mg, suitably 50-100mg, b.i.d. or t.i.d., suitably t.i.d., and the dose of SNRI is in a range selected from 1-200mg,, 1-100mg, 1-50mg, 1-25mg, 10-100mg, 10-50mg or 10-25 mg, suitably 10-100mg, b.i.d or t.i.d, suitably t.i.d.

It will be apparent to the skilled reader that the plasma concentration ranges of the alpha-2-delta ligand and SNRI combinations of the present invention required to provide a therapeutic effect depend on the species to be treated, and components used. For example, for gabapentin in the rat, the Cmax values range from 0.520µg/ml to 10.5µg/ml.

It is possible, using standard PK/PD and allometric methods, to extrapolate the plasma concentration values observed in an animal model to predict the values in a different species, particularly human.

Thus, as a further aspect of the present invention, there is provided a synergistic combination for administration to humans comprising an alpha-2-delta ligand and SNRI, where the plasma concentration range of each component corresponds to the absolute ranges observed in a non-human animal model, preferably the rat model, primarily used to identify a synergistic interaction. Suitably, the plasma concentration range in the human corresponds to a range of 0.05µg/ml to 10.5µg/ml for an alpha-2-delta ligand in the rat model.

Particularly preferred combinations of the invention include those in which each variable of the combination is selected from the suitable parameters for each variable. Even more preferable combinations of the invention include those where each variable of the combination is selected from the more suitable, most suitable, preferred or more preferred parameters for each variable.

### DETAILED DESCRIPTION OF THE INVENTION

The compounds of the present invention are prepared by methods well known to those skilled in the art. Specifically, the patents, patent applications and publications, mentioned hereinabove, exemplify compounds which can be used in the combinations, pharmaceutical compositions, methods and kits in accord with the present invention, and refer to methods of preparing those compounds.

The compounds of the present combination invention can exist in unsolvated forms as well as solvated forms, including hydrated forms. In general, the solvated forms, including hydrated forms, which may contain isotopic substitutions (e.g. D2O, d6-acetone, d6-DMSO), are equivalent to unsolvated forms and are encompassed within the scope of the present invention.

Certain of the compounds of the present invention possess one or more chiral centers and each center may exist in the R or S configuration. The present invention includes all enantiomeric and epimeric forms as well as the appropriate mixtures thereof. Separation of diastereoisomers or cis and trans isomers may be achieved by conventional techniques, e.g. by fractional crystallisation, chromatography or H.P.L.C. of a stereoisomeric mixture of a compound of the invention or a suitable salt thereof.

A number of the alpha-2-delta ligands of the present invention are amino acids. Since amino acids are amphoteric, pharmacologically compatible salts can be salts of appropriate non-toxic inorganic or organic acids or bases. Suitable acid addition salts are the acetate, aspartate, benzoate, besylate, bicarbonate/carbonate, bisulphate, camsylate, citrate, edisylate, esylate, fumarate, gluceptate, gluconate, glucuronate, hibenzate, hydrochloride/chloride, hydrobromide/bromide, hydroiodide/iodide, hydrogen phosphate, isethionate, D- and L-lactate, malate, maleate, malonate, mesylate, methylsulphate, 2-napsylate, nicotinate, nitrate, orotate, palmoate, phosphate, saccharate, stearate, succinate sulphate, D- and L-tartrate, and tosylate salts. Suitable base salts are formed from bases which form non-toxic salts and examples are the sodium, potassium, aluminium, calcium, magnesium, zinc, choline, diolamine, olamine, arginine, glycine, tromethamine, benzathine, lysine, meglumine and diethylamine salts. Salts with quaternary ammonium ions can also be prepared with, for example, the tetramethyl-ammonium ion. The compounds of the invention may also be formed as a zwitterion.

A suitable salt for amino acid compounds of the present invention is the hydrochloride salt. For a review on suitable salts see Stahl and Wermuth, Handbook of Pharmaceutical Salts: Properties, Selection, and Use, Wiley-VCH, Weinheim, Germany (2002).

Also within the scope of the invention are clathrates, drug-host inclusion complexes wherein, in contrast to the aforementioned solvates, the drug and host are present in non-stoichiometric amounts. For a review of such complexes, see J Pharm Sci, 64 (8), 1269-1288 by Haleblian (August 1975).

Hereinafter all references to compounds of the invention include references to salts thereof and to solvates and clathrates of compounds of the invention and salts thereof.

Also included within the present scope of the compounds of the invention are polymorphs thereof.

Prodrugs of the above compounds of the invention are included in the scope of the instant invention. The chemically modified drug, or prodrug, should have a different pharmacokinetic profile to the parent, enabling easier absorption across the mucosal epithelium, better salt formulation and/or solubility, improved systemic stability (for an increase in plasma half-life, for example). These chemical modifications may be
(1) Ester or amide derivatives which may be cleaved by, for example, esterases or lipases. For ester derivatives, the ester is derived from the carboxylic acid moiety of the drug molecule by known means. For amide derivatives, the amide may be derived from the carboxylic acid moiety or the amine moiety of the drug molecule by known means.
(2) Peptides which may be recognized by specific or nonspecific proteinases. A peptide may be coupled to the drug molecule via amide bond formation with the amine or carboxylic acid moiety of the drug molecule by known means.
(3) Derivatives that accumulate at a site of action through membrane selection of a prodrug form or modified prodrug form.
(4) Any combination of 1 to 3.

Aminoacyl-glycolic and -lactic esters are known as prodrugs of amino acids (Wermuth C.G., Chemistry and Industry, 1980:433-435). The carbonyl group of the amino acids can be esterified by known means. Prodrugs and soft drugs are known in the art (Palomino E., Drugs of the Future, 1990;15(4):361-368).

The combination of the present invention is useful for the general treatment of pain, particularly neuropathic pain. Physiological pain is an important protective mechanism designed to warn of danger from potentially injurious stimuli from the external environment. The system operates through a specific set of primary sensory neurones and is exclusively activated by noxious stimuli via peripheral transducing mechanisms (Millan 1999 Prog. Neurobio. 57: 1-164 for an integrative Review). These sensory fibres are known as nociceptors and are characterised by small diameter axons with slow conduction velocities. Nociceptors encode the intensity, duration and quality of noxious stimulus and by virtue of their topographically organised projection to the spinal cord, the location of the stimulus. The nociceptors are found on nociceptive nerve fibres of which there are two main types, A-delta fibres (myelinated) and C fibres (non-myelinated). The activity generated by nociceptor input is transferred after complex processing in the dorsal horn, either directly or via brain stem relay nuclei to the ventrobasal thalamus and then on to the cortex, where the sensation of pain is generated.

Intense acute pain and chronic pain may involve the same pathways driven by pathophysiological processes and as such cease to provide a protective mechanism and instead contribute to debilitating symptoms associated with a wide range of disease states. Pain is a feature of many trauma and disease states. When a substantial injury, via disease or trauma, to body tissue occurs the characteristics of nociceptor activation are altered. There is sensitisation in the periphery, locally around the injury and centrally where the nociceptors terminate. This leads to hypersensitivity at the site of damage and in nearby normal tissue. In acute pain these mechanisms can be useful and allow for the repair processes to take place and the hypersensitivity returns to normal once the injury has healed. However, in many chronic pain states, the hypersensitivity far outlasts the healing process and is normally due to nervous system injury. This injury often leads to maladaptation of the afferent fibres (Woolf & Salter 2000 Science 288: 1765-1768). Clinical pain is present when discomfort and abnormal sensitivity feature among the patient's symptoms. Patients tend to be quite heterogeneous and may present with various pain symptoms. There are a number of typical pain subtypes: 1) spontaneous pain which may be dull, burning, or stabbing; 2) pain responses to noxious stimuli are exaggerated (hyperalgesia); 3) pain is produced by normally innocuous stimuli (allodynia) (Meyer et al., 1994 Textbook of Pain 13-44). Although patients with back pain, arthritis pain, CNS trauma, or neuropathic pain may have similar symptoms, the underlying mechanisms are different and, therefore, may require different treatment strategies. Therefore pain can be divided into a number of different areas because of differing pathophysiology, these include nociceptive, inflammatory, neuropathic pain etc. It should be noted that some types of pain have multiple aetiologies and thus can be classified in more than one area, e.g. Back pain, Cancer pain have both nociceptive and neuropathic components.

Nociceptive pain is induced by tissue injury or by intense stimuli with the potential to cause injury. Pain afferents are activated by transduction of stimuli by nociceptors at the site of injury and sensitise the spinal cord at the level of their termination. This is then relayed up the spinal tracts to the brain where pain is perceived (Meyer et al., 1994 Textbook of Pain 13-44). The activation of nociceptors activates two types of afferent nerve fibres. Myelinated A-delta fibres transmitted rapidly and are responsible for the sharp and stabbing pain sensations, whilst unmyelinated C fibres transmit at a slower rate and convey the dull or aching pain. Moderate to severe acute nociceptive pain is a prominent feature of, but is not limited to pain from strains/sprains, post-operative pain (pain following any type of surgical procedure), posttraumatic pain, bums, myocardial infarction, acute pancreatitis, and renal colic. Also cancer related acute pain syndromes commonly due to therapeutic interactions such as chemotherapy toxicity, immunotherapy, hormonal therapy and radiotherapy. Moderate to severe acute nociceptive pain is a prominent feature of, but is not limited to, cancer pain which may be tumour related pain, (e.g. bone pain, headache and facial pain, viscera pain) or associated with cancer therapy (e.g. postchemotherapy syndromes, chronic postsurgical pain syndromes, post radiation syndromes), back pain which may be due to herniated or ruptured intervertabral discs or abnormalities of the lumber facet joints, sacroiliac joints, paraspinal muscles or the posterior longitudinal ligament

Neuropathic pain is defined as pain initiated or caused by a primary lesion or dysfunction in the nervous system (IASP definition). Nerve damage can be caused by trauma and disease and thus the term 'neuropathic pain' encompasses many disorders with diverse aetiologies. These include but are not limited to, Diabetic neuropathy, Post herpetic neuralgia, Back pain, Cancer neuropathy, HIV neuropathy, Phantom limb pain, Carpal Tunnel Syndrome, chronic alcoholism, hypothyroidism, trigeminal neuralgia, uremia, or vitamin deficiencies. Neuropathic pain is pathological as it has no protective role. It is often present well after the original cause has dissipated, commonly lasting for years, significantly decreasing a patients quality of life (Woolf and Mannion 1999 Lancet 353: 1959-1964). The symptoms of neuropathic pain are difficult to treat, as they are often heterogeneous even between patients with the same disease (Woolf & Decosterd 1999 Pain Supp. 6: S141-S147; Woolf and Mannion 1999 Lancet 353: 1959-1964). They include spontaneous pain, which can be continuous, or paroxysmal and abnormal evoked pain, such as hyperalgesia (increased sensitivity to a noxious stimulus) and allodynia (sensitivity to a normally innocuous stimulus).

The inflammatory process is a complex series of biochemical and cellular events activated in response to tissue injury or the presence of foreign substances, which result in swelling and pain (Levine and Taiwo 1994: Textbook of Pain 45-56). Arthritic pain makes up the majority of the inflammatory pain population. Rheumatoid disease is one of the commonest chronic inflammatory conditions in developed countries and rheumatoid arthritis is a common cause of disability. The exact aetiology of RA is unknown, but current hypotheses suggest that both genetic and microbiological factors may be important (Grennan & Jayson 1994 Textbook of Pain 397-407). It has been estimated that almost 16 million Americans have symptomatic osteoarthritis (OA) or degenerative joint disease, most of whom are over 60 years of age, and this is expected to increase to 40 million as the age of the population increases, making this a public health problem of enormous magnitude (Houge & Mersfelder 2002 Ann Pharmacother. 36: 679-686; McCarthy et al., 1994 Textbook of Pain 387-395). Most patients with OA seek medical attention because of pain. Arthritis has a significant impact on psychosocial and physical function and is known to be the leading cause of disability in later life. Other types of inflammatory pain include but are not limited to inflammatory bowel diseases (IBD).

Other types of pain include but are not limited to;
- Musculo-skeletal disorders including but not limited to myalgia, fibromyalgia, spondylitis, sero-negative (non-rheumatoid) arthropathies, non-articular rheumatism, dystrophinopathy, Glycogenolysis, polymyositis, pyomyositis.
- Central pain or 'thalamic pain' as defined by pain caused by lesion or dysfunction of the nervous system including but not limited to central post-stroke pain, multiple sclerosis, spinal cord injury, Parkinson's disease and epilepsy.
- Heart and vascular pain including but not limited to angina, myocardical infarction, mitral stenosis, pericarditis, Raynaud's phenomenon, scleredoma, scleredoma, skeletal muscle ischemia.
- Visceral pain, and gastrointestinal disorders. The viscera encompasses the organs of the abdominal cavity. These organs include the sex organs, spleen and part of the digestive system. Pain associated with the viscera can be divided into digestive visceral pain and non-digestive visceral pain. Commonly encountered gastrointestinal (GI) disorders include the functional bowel disorders (FBD) and the inflammatory bowel diseases (IBD). These GI disorders include a wide range of disease states that are currently only moderately controlled, including - for FBD, gastro-esophageal reflux, dyspepsia, the irritable bowel syndrome (IBS) and functional abdominal pain syndrome (FAPS), and - for IBD, Crohn's disease, ileitis, and ulcerative colitis, which all regularly produce visceral pain. Other types of visceral pain include the pain associated with dysmenorrhea, pelvic pain, cystitis and pancreatitis.
- Head pain including but not limited to migraine, migraine with aura, migraine without aura cluster headache, tension-type headache.
- Orofacial pain including but not limited to dental pain, temporomandibular myofascial pain.

The combination of the present invention is also useful in the treatment of urinary incontinence, such as genuine stress incontinence (GSI), stress urinary incontinence (SUI) or urinary incontinence in the elderly; overactive bladder (OAB), including idiopathic detrusor instability, detrusor overactivity secondary to neurological diseases (e.g. Parkinson's disease, multiple sclerosis, spinal cord injury and stroke) and detrusor overactivity secondary to bladder outflow obstruction (e.g. benign prostatic hyperplasia (BPH), urethral stricture or stenosis); nocturnal enuresis; urinary incontinence due to a combination of the above conditions (e.g. genuine stress incontinence associated with overactive bladder); and urinary symptoms, such as frequency and urgency.

The combination is also useful in the treatment of faecal incontinence.

As a yet further aspect, there is provided the use of an alpha-2-delta ligand and SNRI, with the proviso that the compounds (i)-(xxv) of WO02/85839 in combination with a serotonin reuptake inhibitor, particularly fluoxetine, paroxetine, citalopram and sertraline, a mixed serotonin-noradrenaline reuptake inhibitor, paticularly milnacipran, venlafaxine and duloxetine, and a noradrenaline reuptake inhibitor, particularly reboxetine are excluded, in the manufacture of a medicament for the curative, prophylactic or palliative treatment of pain, particularly neuropathic pain.

As an alternative feature, the invention provides the use of a synergistic effective amount of an alpha-2-delta ligand and SNRI in the manufacture of a medicament for the curative, prophylactic or palliative treatment of pain, particularly neuropathic pain.

As an alternative aspect, there is provided a curative, prophylactic or palliative treatment of pain, particularly neuropathic pain, comprising simultaneous, sequential or separate administration of a therapeutically effective amount of an alpha-2-delta ligand and SNRI, to a mammal in need of said treatment, with the proviso that the combinations disclosed in WO02/85839, i.e. a compound of formula (i)-(xxv) in combination with: serotonin reuptake inhibitors, e.g. fluoxetine, paroxetine, citalopram and sertraline; mixed serotonin-noradrenaline reuptake inhibitors, e.g. milnacipran, venlafaxine and duloxetine; or noradrenaline reuptake inhibitors, e.g. reboxetine are excluded.

As an alternative feature, there is provided a curative, prophylactic or palliative treatment of pain, particularly neuropathic pain, comprising simultaneous, sequential or separate administration of a therapeutically synergistic amount of an alpha-2-delta ligand and SNRI, to a mammal in need of said treatment.

The biological activity of the alpha-2-delta ligands of the invention may be measured in a radioligand binding assay using [³H]gabapentin and the α₂δ subunit derived from porcine brain tissue (Gee N.S., Brown J.P., Dissanayake V.U.K., Offord J., Thurlow R., Woodruff G.N., J. Biol. Chem., 1996;271:5879-5776). Results may be expressed in terms of µM or nM α2δ binding affinity.

The ability of compounds to act as selective serotonin reuptake inhibitiors can be measured in vivo according to established procedures, e.g. according to Example 68 of US4536518.

The ability of compounds to act as dual serotonin-noradrenaline or selective noradrenaline reuptake inhibitors can be measured according to established procedures, particularly in the documents mentioned hereinabove.

The elements of the combination of the instant invention may be administered separately, simultaneously or sequentially for the treatment of pain. The combination may also optionally be administered with one or more other pharmacologically active agents. Suitable optional agents include:
(i) opioid analgesics, e.g. morphine, heroin, hydromorphone, oxymorphone, levorphanol, levallorphan, methadone, meperidine, fentanyl, cocaine, codeine, dihydrocodeine, oxycodone, hydrocodone, propoxyphene, nalmefene, nalorphine, naloxone, naltrexone, buprenorphine, butorphanol, nalbuphine and pentazocine;
(ii) nonsteroidal antiinflammatory drugs (NSAIDs), e.g. aspirin, diclofenac, diflusinal, etodolac, fenbufen, fenoprofen, flufenisal, flurbiprofen,ibuprofen, indomethacin, ketoprofen, ketorolac, meclofenamic acid, mefenamic acid, nabumetone, naproxen, oxaprozin, phenylbutazone, piroxicam, sulindac, tolmetin, zomepirac, and their pharmaceutically acceptable salts;
(iii) barbiturate sedatives, e.g. amobarbital, aprobarbital, butabarbital, butabital, mephobarbital, metharbital, methohexital, pentobarbital, phenobartital, secobarbital, talbutal, theamylal, thiopental and their pharmaceutically acceptable salts;
(iv) benzodiazepines having a sedative action, e.g. chlordiazepoxide, clorazepate, diazepam, flurazepam, lorazepam, oxazepam, temazepam, triazolam and their pharmaceutically acceptable salts,
(v) H₁ antagonists having a sedative action, e.g. diphenhydramine, pyrilamine, promethazine, chlorpheniramine, chlorcyclizine and their pharmaceutically acceptable salts;
(vi) miscellaneous sedatives such as glutethimide, meprobamate, methaqualone, dichloralphenazone and their pharmaceutically acceptable salts;
(vii) skeletal muscle relaxants, e.g. baclofen, carisoprodol, chlorzoxazone, cyclobenzaprine, methocarbamol, orphrenadine and their pharmaceutically acceptable salts,
(viii) NMDA receptor antagonists, e.g. dextromethorphan ((+)-3-hydroxy-N-methylmorphinan) and its metabolite dextrorphan ((+)-3-hydroxy-N-methylmorphinan), ketamine, memantine, pyrroloquinoline quinone and cis-4-(phosphonomethyl)-2- piperidinecarboxylic acid and their pharmaceutically acceptable salts;
(ix) alpha-adrenergic active compounds, e.g. doxazosin, tamsulosin, clonidine and 4-amino-6,7-dimethoxy-2-(5-methanesulfonamido-1,2,3,4-tetrahydroisoquinol-2-yl)-5-(2-pyridyl) quinazoline;
(x) tricyclic antidepressants, e.g. desipramine, imipramine, amytriptiline and nortriptiline;
(xi) anticonvulsants, e.g. carbamazepine and valproate;
(xii) Tachykinin (NK) antagonists, particularly Nk-3, NK-2 and NK-1 e.g. antagonists, (αR,9R)-7-[3,5-bis(trifluoromethyl)benzyl]-8,9,10,11-tetrahydro-9-methyl-5-(4-methylphenyl)-7H-[1,4]diazocino[2,1-g][1,7]naphthridine-6-13-dione (TAK-637), 5-[[(2R,3S)-2-[(1R)-1-[3,5-bis(trifluoromethyl)phenyl]ethoxy-3-(4-fluorophenyl)-4-morpholinyl]methyl]-1,2-dihydro-3H-1,2,4-triazol-3-one (MK-869), lanepitant, dapitant and 3-[[2-methoxy-5-(trifluoromethoxy)phenyl]methylamino]-2-phenyl-piperidine (2S,3S)
(xiii) Muscarinic antagonists, e.g oxybutin, tolterodine, propiverine, tropsium chloride and darifenacin;
(xiv) COX-2 inhibitors, e.g. celecoxib, rofecoxib and valdecoxib;
(xv) Non-selective COX inhibitors (preferably with GI protection), e.g. nitroflurbiprofen (HCT-1026);
(xvi) coal-tar analgesics, in particular, paracetamol;
(xvii) neuroleptics, such as droperidol;
(xviii) Vanilloid receptor agonists, e.g. resinferatoxin;
(xix) Beta-adrenergic compounds such as propranolol;
(xx) Local anaesthetics, such as mexiletine;
(xxi) Corticosteriods, such as dexamethasone
(xxii) serotonin receptor agonists and antagonists;
(xxiii) cholinergic (nicotinic) analgesics;
(xxiv) miscellaneous agents such as Tramadol®;
(xxv) PDEV inhibitors, such as sildenafil, vardenafil or taladafil.

The present invention extends to a product comprising an alpha-2-delta ligand and SNRI and one or more other therapeutic agents, such as those listed above, for simultaneous, separate or sequential use in the curative, prophylactic treatment of pain, particularly neuropathic pain.

The combination of the invention can be administered alone but one or both elements will generally be administered in an admixture with suitable pharmaceutical excipient(s), diluent(s) or carrier(s) selected with regard to the intended route of administration and standard pharmaceutical practice. If appropriate, auxiliaries can be added. Auxiliaries are preservatives, anti-oxidants, flavours or colourants. The compounds of the invention may be of immediate-, delayed-, modified-, sustained-, pulsed- or controlled-release type.

The elements of the combination of the present invention can be administered, for example but not limited to, the following route: orally, buccally or sublingually in the form of tablets, capsules, multi-and nano-particulates, gels, films (incl. muco-adhesive), powder, ovules, elixirs, lozenges (incl. liquid-filled), chews, solutions, suspensions and sprays. The compounds of the invention may also be administered as osmotic dosage form, or in the form of a high energy dispersion or as coated particles or fast-dissolving, fast -disintegrating dosage form as described in Ashley Publications, 2001 by Liang and Chen. The compounds of the invention may be administered as crystalline or amorphous products, freeze dried or spray dried. Suitable formulations of the compounds of the invention may be in hydrophilic or hydrophobic matrix, ion-exchange resin complex, coated or uncoated form and other types as described in US 6,106,864 as desired. Such pharmaceutical compositions, for example, tablets, may contain excipients such as microcrystalline cellulose, lactose, sodium citrate, calcium carbonate, dibasic calcium phosphate, glycine and starch (preferably corn, potato or tapioca starch), mannitol, disintegrants such as sodium starch glycolate, crosscarmellose sodium and certain complex silicates, and granulation binders such as polyvinylpyrrolidone, hydroxypropylmethylcellulose (HPMC), triglycerides, hydroxypropylcellulose (HPC), bentonite sucrose, sorbitol, gelatin and acacia. Additionally, lubricating agents may be added to solid compositions such as magnesium stearate, stearic acid, glyceryl behenate, PEG and talc or wetting agents, such as sodium lauryl sulphate. Additionally, polymers such as carbohydrates, phospoholipids and proteins may be included.

Fast dispersing or dissolving dosage fromulations (FDDFs) may contain the following ingredients: aspartame, acesulfame potassium, citric acid, croscarmellose sodium, crospovidone, diascorbic acid, ethyl acrylate, ethyl cellulose, gelatin, hydroxypropylmethyl cellulose, magnesium stearate, mannitol, methyl methacrylate, mint flavouring, polyethylene glycol, fumed silica, silicon dioxide, sodium starch glycolate, sodium stearyl fumarate, sorbitol or xylitol. The terms dispersing or dissolving as used herein to describe FDDFs are dependent upon the solubility of the drug substance used, i.e. where the drug substance is insoluble a fast dispersing dosage form can be prepared and where the drug substance is soluble a fast dissolving dosage form can be prepared.

The solid dosage form, such as tablets are manufactured by a standard process, for example, direct compression or a wet, dry or melt granulation, melt congealing and extrusion process. The tablet cores which may be mono or multi-layer may be coated with appropriate overcoats known in the art.

Solid compositions of a similar type may also be employed as fillers in capsules such as gelatin, starch or HPMC capsules. Preferred excipients in this regard include lactose, starch, a cellulose, milk sugar or high molecular weight polyethylene glycols. Liquid compositions may be employed as fillers in soft or hard capsules such as gelatin capsule. For aqueous and oily suspensions, solutions, syrups and/or elixirs, the compounds of the invention may be combined with various sweetening or flavouring agents, colouring matter or dyes, with emulsifying and/or suspending agents and with diluents such as water, ethanol, propylene glycol, methylcellulose, alginic acid or sodium alginate, glycerin, oils, hydrocolloid agents and combinations thereof. Moreover, formulations containing these compounds and excipients may be presented as a dry product for constitution with water or other suitable vehicles before use.

Liquid form preparations include solutions, suspensions, and emulsions, for example, water or water propylene glycol solutions. For parenteral injection, liquid preparations can be formulated in solution in aqueous polyethylene glycol solution. Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

The elements of the combination of the present invention can also be administered by injection, that is, intravenously, intramuscularly, intracutaneously, intraduodenally, or intraperitoneally, intraarterially, intrathecally, intraventricularly, intraurethrally, intrasternally, intracranially, intraspinally or subcutaneously, or they may be administered by infusion, needle-free injectors or implant injection techniques. For such parenteral administration they are best used in the form of a sterile aqueous solution, suspension or emulsion (or system so that can include micelles) which may contain other substances known in the art, for example, enough salts or carbohydrates such as glucose to make the solution isotonic with blood. The aqueous solutions should be suitably buffered (preferably to a pH of from 3 to 9), if necessary. For some forms of parenteral administration they may be used in the form of a sterile non-aqueous system such as fixed oils, including mono- or diglycerides, and fatty acids, including oleic acid. The preparation of suitable parenteral formulations under sterile conditions for example lyophilisation is readily accomplished by standard pharmaceutical techniques well-known to those skilled in the art. Alternatively, the active ingredient may be in powder form for constitution with a suitable vehicle (e.g. sterile, pyrogen-free water) before use.

Also, the elements of the combination of the present invention can be administered intranasally or by inhalation. They are conveniently delivered in the form of a dry powder (either alone, as a mixture, for example a dry blend with lactose, or a mixed component particle, for example with phospholipids) from a dry powder inhaler or an aerosol spray presentation from a pressurised container, pump, spray, atomiser (preferably an atomiser using electrohydrodynamics to produce a fine mist) or nebuliser, with or without the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide, a further perfluorinated hydrocarbon such as Perflubron (trade mark) or other suitable gas. In the case of a pressurised aerosol, the dosage unit may be determined by providing a valve to deliver a metered amount. The pressurised container, pump, spray, atomiser or nebuliser may contain a solution or suspension of the active compound, e.g. using a mixture of ethanol (optionally, aqueous ethanol) or a suitable agent for dispersing, solubilising or extending release and the propellant as the solvent, which may additionally contain a lubricant, e.g. sorbitan trioleate. Capsules, blisters and cartridges (made, for example, from gelatin or HPMC) for use in an inhaler or insufflator may be formulated to contain a powder mix of the compound of the invention, a suitable powder base such as lactose or starch and a performance modifier such as l-leucine, mannitol or magnesium stearate.

Prior to use in a dry powder formulation or suspension formulation for inhalation the elements of the combination of the invention will be micronised to a size suitable for delivery by inhalation (typically considered as less than 5 microns). Micronisation could be achieved by a range of methods, for example spiral jet milling, fluid bed jet milling, use of supercritical fluid crystallisation or by spray drying.

A suitable solution formulation for use in an atomiser using electrohydrodynamics to produce a fine mist may contain from 1µg to 10mg of the compound of the invention per actuation and the actuation volume may vary from 1 to 100µl. A typical formulation may comprise the elements of the combination of the invention, propylene glycol, sterile water, ethanol and sodium chloride. Alternative solvents may be used in place of propylene glycol, for example glycerol or polyethylene glycol.

Alternatively, the elements of the combination of the invention may be administered topically to the skin, mucosa, dermally or transdermally, for example, in the form of a gel, hydrogel, lotion, solution, cream, ointment, dusting powder, dressing, foam, film, skin patch, wafers, implant, sponges, fibres, bandage, microemulsions and combinations thereof. For such applications, the compounds of the invention can be suspended or dissolved in, for example, a mixture with one or more of the following: mineral oil, liquid petrolatum, white petrolatum, propylene glycol, polyoxyethylene polyoxypropylene compound, emulsifying wax , fixed oils, including synthetic mono- or diglycerides, and fatty acids, including oleic acid, water, sorbitan monostearate, a polyethylene glycol, liquid paraffin, polysorbate 60, cetyl esters wax, cetearyl alcohol, 2-octyldodecanol, benzyl alcohol, alcohols such as ethanol. Alternatively, penetration enhancers may be used. The following may also be used polymers, carbohydrates, proteins, phospolipids in the form of nanoparticles (such as niosomes or liposomes) or suspended or dissolved. In addition, they may be delivered using iontophoresis, electroporation, phonophoresis and sonophoresis.

Alternatively, the elements of the combination of the invention can be administered rectally, for example in the form of a suppository or pessary. They may also be administered by vaginal route. For example, these compositions may be prepared by mixing the drug with a suitable non-irritant excipients, such as cocoa butter, synthetic glyceride esters or polyethylene glycols, which are solid at ordinary temperatures, but liquefy and/or dissolve in the cavity to release the drug.

The elements of the combination of the invention may also be administered by the ocular route. For ophthalmic use, the compounds can be formulated as micronised suspensions in isotonic, pH adjusted, sterile saline, or, preferably, as solutions in isotonic, pH adjusted, sterile saline. A polymer may be added such as crossed-linked polyacrylic acid, polyvinylalcohol, hyaluronic acid, a cellulosic polymer (e.g. hydroxypropylmethylcellulose, hydroxyethylcellulose, methyl cellulose), or a heteropolysaccharide polymer (e.g. gelan gum). Alternatively, they may be formulated in an ointment such as petrolatum or mineral oil, incorporated into bio-degradable (e.g. absorbable gel sponges, collagen) or non-biodegradable (e.g. silicone) implants, wafers, drops, lenses or delivered via particulate or vesicular systems such as niosomes or liposomes. Formulations may be optionally combined with a preservative, such as benzalkonium chloride. In addition, they may be delivered using iontophoresis. They may also be administered in the ear, using for example but not limited to the drops.

The elements of the combination of the invention may also be used in combination with a cyclodextrin. Cyclodextrins are known to form inclusion and non-inclusion complexes with drug molecules. Formation of a drug-cyclodextrin complex may modify the solubility, dissolution rate, taste-masking, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

The term 'administered' includes delivery by viral or non-viral techniques. Viral delivery mechanisms include but are not limited to adenoviral vectors, adeno- associated viral (AAV) vectors, herpes viral vectors, retroviral vectors, lentiviral vectors, and baculoviral vectors. Non-viral delivery mechanisms include lipid mediated transfection, lipsomes, immunoliposomes, lipofectin, cationic facial amphiphiles (CFAs) and combinations thereof. The routes for such delivery mechanisms include but are not limited to mucosal, nasal, oral, parenteral, gastrointestinal, topical or sublingual routes.

Thus, as a further aspect of the present invention, there is provided a pharmaceutical composition comprising a combination comprising an alpha-2-delta ligand and SNRI, or pharmaceutically acceptable salts thereof, with the proviso that the compounds (i)-(xxv) of WO02/85839 in combination with a serotonin reuptake inhibitor, particularly fluoxetine, paroxetine, citalopram and sertraline, a mixed serotonin-noradrenaline reuptake inhibitor, paticularly milnacipran, venlafaxine and duloxetine, and a noradrenaline reuptake inhibitor, particularly reboxetine are excluded, and a suitable excipient, diluent or carrier. Suitably, the composition is suitable for use in the treatment of pain, particularly neuropathic pain.

As an aspect of the present invention, there is provided a pharmaceutical composition comprising a synergistic combination comprising an alpha-2-delta ligand and SNRI, or pharmaceutically acceptable salts thereof, and a suitable excipient, diluent or carrier. Suitably, the composition is suitable for use in the treatment of pain, particularly neuropathic pain.

For non-human animal administration, the term 'pharmaceutical' as used herein may be replaced by 'veterinary.'

The element of the pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsules, tablet, cachet, or lozenge itself, or it can be the appropriate number of any of these in packaged form. The quantity of active component in a unit dose preparation may be varied or adjusted from 0.1 mg to 1 g according to the particular application and the potency of the active components. In medical use the drug may be administered three times daily as, for example, capsules of 100 or 300 mg. In therapeutic use, the compounds utilized in the pharmaceutical method of this invention are administered at the initial dosage of about 0.01 mg to about 100 mg/kg daily. A daily dose range of about 0.01 mg to about 100 mg/kg is preferred. The dosages, however, may be varied depending upon the requirements of the patient, the severity of the condition being treated, and the compounds being employed. Determination of the proper dosage for a particular situation is within the skill of the art. Generally, treatment is initiated with smaller dosages which are less than the optimum dose of the compounds. Thereafter, the dosage is increased by small increments until the optimum effect under the circumstances is reached. For convenience, the total daily dosage may be divided and administered in portions during the day, if desired.

For veterinary use, a combination according to the present invention or veterinarily acceptable salts or solvates thereof, is administered as a suitably acceptable formulation in accordance with normal veterinary practice and the veterinary surgeon will determine the dosing regimen and route of administration which will be most appropriate for a particular animal.

### BIOLOGY EXAMPLES

### METHODS

### Animals

Male Sprague Dawley rats (200-250g), obtained from Charles River, (Margate, Kent, U.K.) were housed in groups of 6. All animals were kept under a 12h light/dark cycle (lights on at 07h 00min) with food and water *ad libitum*. All experiments were carried out by an observer unaware of drug treatments.

### CCI surgery in the rat

Animals were anaesthetised with isoflurane. The sciatic nerve was ligated as previously described by Bennett and Xie, 1988. Animals were placed on a homeothermic blanket for the duration of the procedure. After surgical preparation the common sciatic nerve was exposed at the middle of the thigh by blunt dissection through biceps femoris. Proximal to the sciatic trifurcation, about 7mm of nerve was freed of adhering tissue and 4 ligatures (4-0 silk) were tied loosely around it with about 1mm spacing. The incision was closed in layers and the wound treated with topical antibiotics.

### Effect of combinations on the maintenance of CCI-induced static and dynamic allodynia

Dose-responses to gabapentin and SNRI were first performed alone in the CCI model. Combinations were examined following a fixed ratio design. A dose-response to each fixed dose ratio of the combination was performed. On each test day, baseline paw withdrawal thresholds (PWT) to von Frey hairs and paw withdrawal latencies (PWL) to a cotton bud stimulus were determined prior to drug treatment.

### Evaluation of allodynia

Static allodynia was measured using Semmes-Weinstein von Frey hairs (Stoelting, Illinois, U.S.A.). Animals were placed into wire mesh bottom cages allowing access to the underside of their paws. Animals were habituated to this environment prior to the start of the experiment. Static allodynia was tested by touching the plantar surface of the animals right hind paw with von Frey hairs in ascending order of force ( 0.7, 1.2, 1.5, 2, 3.6, 5.5, 8.5, 11.8, 15.1 and 29g) for up to 6sec. Once a withdrawal response was established, the paw was retested, starting with the next descending von Frey hair until no response occurred. The highest force of 29g lifted the paw as well as eliciting a response, thus represented the cut off point. The lowest amount of force required to elicit a response was recorded as the PWT in grams.

Dynamic allodynia was assessed by lightly stroking the plantar surface of the hind paw with a cotton bud. Care was taken to perform this procedure in fully habituated rats that were not active to avoid recording general motor activity. At least three measurements were taken at each time point the mean of which represented the paw withdrawal latency (PWL). If no reaction was exhibited within 15s the procedure was terminated and animals were assigned this withdrawal time. Thus 15s effectively represents no withdrawal. A withdrawal response was often accompanied with repeated flinching or licking of the paw. Dynamic allodynia was considered to be present if animals responded to the cotton stimulus before 8s of stroking.

### Combination studies

Dose responses are first performed to both the alpha-2-delta ligand (p.o.) and/or SNRI (s.c. or p.o.) alone. A number of fixed dose ratios of the combination may then be examined. Dose responses to each fixed dose ratio were performed with the time-course for each experiment determined by the duration of antiallodynic-action of each separate ratio. Various fixed dose ratios of the combinations by weight may be examined.

Suitable SNRI compounds of the present invention may be prepared as described in the references or are obvious to those skilled in the art on the basis of these documents.

Suitable alpha-2-delta ligand compounds of the present invention may be prepared as described herein below or in the aforementioned patent literature references, which are illustrated by the following non-limiting examples and intermediates.

Chemistry Examples Examples not referring to gabapentin, pregabalin or (S,S)-reboxetine are not part of the invention as claimed and are to be regarded as illustrative only.

Example 1. (3S,5R)-3-Amino-5-methyl-octanoic acid hydrochloride(R)-2,6-Dimethyl-non-2-ene. To (*S*)-citronellyl bromide (50 g, 0.228 mol) in THF (800 mL) at 0°C was added LiCl (4.3 g) followed by CuCl₂ (6.8 g). After 30 minutes methylmagnesium chloride (152 mL of a 3 M solution in THF, Aldrich) was added and the solution warmed to room temperature. After 10 hours the solution was cooled to 0°C and a saturated aqueous solution of ammonium chloride carefully added. The resultant two layers were separated and the aqueous phase extracted with ether. The combined organic phases were dried (MgSO₄) and concentrated to give (R)-2,6-dimethyl-non-2-ene. 32.6 g; 93%. Used without further purification. ¹H NMR (400 MHz; CDCl₃) δ 5.1 (m, 1H), 1.95 (m, 2H), 1.62 (s, 3H), 1.6 (s, 3H), 1.3 (m, 4H), 1.2 (m, 2H), 0.8 (s, 6H).

(R)-4-Methyl-heptanoic acid. To (R)-2,6-dimethyl-non-2-ene (20 g, 0.13 mol) in acetone (433 mL) was added a solution of CrO₃ (39 g, 0.39 mol) in H₂SO₄ (33 mL)/H₂O (146 mL) over 50 minutes. After 6 hours a further amount of CrO₃ (26 g, 0.26 mol) in H₂SO₄ (22 mL)/H₂O (100 mL) was added. After 12 hours the solution was diluted with brine and the solution extracted with ether. The combined organic phases were dried (MgSO₄) and concentrated. Flash chromatography (gradient of 6:1 to 2:1 hexane/EtOAc) gave (R)-4-methyl-heptanoic acid as an oil. 12.1 g; 65%. MS, *m*/*z* (relative intensity): 143 [M-H, 100%].

(4R,5S)-4-Methyl-3-((R)-4-methyl-heptanoyl)-5-phenyl-oxazolidin-2-one. To (R)-4-methyl-heptanoic acid (19 g, 0.132 mol) and triethylamine (49.9 g, 0.494 mol) in THF (500 mL) at 0°C was added trimethylacetylchloride (20 g, 0.17 mol). After 1 hour LiCl (7.1 g, 0.17 mol) was added followed by (4R,5S)-(+)-4-methyl-5-phenyl-2-oxazolidinone) **3** (30 g, 0.17 mol). The mixture was warmed to room temperature and after 16 hours the filtrate was removed by filtration and the solution concentrated under reduced pressure. Flash chromatography (7:1 hexane/EtOAc) gave (4R,5S)-4-methyl-3-((R)-4-methyl-heptanoyl)-5-phenyl-oxazolidin-2-one as an oil. 31.5 g; 79%. [α]_{D} = +5.5 (c 1 in CHCl₃). MS, *m*/*z* (relative intensity): 304 [M+H, 100%].

(3S,5R)-5-Methyl-3-((4R,5S)-4-methyl-2-oxo-5-phenyl-oxazolidine-3-carbonyl)-octanoic acid tert-butyl ester. To (4R,5S)-4-methyl-3-((R)-4-methyl-heptanoyl)-5-phenyl-oxazolidin-2-one (12.1 g, 0.04 mol) in THF (200 ml) at -50°C was added sodium bis(trimethylsilyl)amide (48 mL of a 1 M solution in THF). After 30 min t-butylbromoaceate (15.6 g, 0.08 mol) was added. The solution was stirred for 4 hours at -50°C and then warmed to room temperature. After 16 hours a saturated aqueous solution of ammonium chloride was added and the two layers separated. The aqueous phase was extracted with ether and the combined organic phases dried (MgSO₄) and concentrated. Flash chromatography (9:1 hexane/EtOAc) gave (3S,5R)-5-methyl-3-((4R,5S)-4-methyl-2-oxo-5-phenyl-oxazolidine-3-carbonyl)-octanoic acid tert-butyl ester as a white solid 12 g; 72%. [α]_{D} = +30.2 (c 1 in CHCl₃). ¹³C NMR (100 MHz; CDCl₃) δ 176.47, 171.24, 152.72, 133.63, 128.87, 125.86, 80.85, 78.88, 55.34, 39.98, 38.77, 38.15, 37.58, 30.60, 28.23, 20.38, 20.13, 14.50, 14.28.

(S)-2-((R)-2-Methyl-pentyl)-succinic acid 4-tert-butyl ester. To (3S,5R)-5-methyl-3-((4R,5S)-4-methyl-2-oxo-5-phenyl-oxazolidine-3-carbonyl)-octanoic acid tert-butyl ester (10.8 g, 0.025 mol) in H₂O (73 mL) and THF (244 mL) at 0°C was added a premixed solution of LiOH (51.2 mL of a 0.8 M solution) and H₂O₂ (14.6 mL of a 30% solution). After 4 hours a further 12.8 mL LiOH (0.8 M solution) and 3.65 mL of H₂O₂ (30% solution) was added. After 30 minutes sodium bisulfite (7 g), sodium sulfite (13 g), and water (60 mL) was added followed by hexane (100 mL) and ether (100 mL). The two layers were separated and the aqueous layer extracted with ether. The combined organic phases were concentrated to an oil that was dissolved in heptane (300 mL). The resultant solid was filtered off and the filtrate dried (MgSO₄) and concentrated to afford (S)-2-((R)-2-methyl-pentyl)-succinic acid 4-tert-butyl ester (6 g, 93%) which was used immediately without further purification. MS, *m*/*z* (relative intensity): 257 [M+H, 100%].

(3S, 5R)-3-Benzyoxycarbonylamino-5-methyl-octanoic acid, *tert*-butyl ester. A solution of (S)-2-((R)-2-methyl-pentyl)-succinic acid 4-tert-butyl ester (6.0 g, 23.22 mmol) and triethylamine (3.64 mL, 26.19 mmol) in toluene (200 mL) was treated with diphenylphosphoryl azide (5.0 mL, 23.22 mL) and stirred at room temperature for 0.5 hours. After the reaction mixture was then heated at reflux for 3h and cooled briefly, benzyl alcohol was added (7.2 mL, 69.7 mmol) and the solution heated for another 3 h. After the reaction mixture was allowed to cool, it was diluted with ethyl ether (200 mL) and the combined organic layer was washed successively with saturated NaHCO₃ and brine and dried (Na₂SO₄). The concentrated organic component was purified by chromatography (MPLC) eluting with 8:1 hexanes: ethyl acetate to provide (3S, 5R)-3-benzyoxycarbonylamino-5-methyl-octanoic acid, *tert*-butyl ester (6.4 g, 75.8%). MS: M+1: 364.2, 308.2.

(3S, 5R)-3-Amino-5-methyl-octanoic acid, *tert*-butyl ester. A solution of (3S, 5R)-3-benzyoxycarbonylamino-5-methyl-octanoic acid, *tert*-butyl ester (2.14g, 5.88 mmol) in THF (50 mL) was treated with Pd/C (0.2 g) and H₂ at 50 psi for 2 hours. The reaction mixture was then filtered and concentrated to an oil in vacuo to give (3S, 5R)-3-amino-5-methyl-octanoic acid, *tert*-butyl ester in quantitative yield. MS: M+1: 230.2, 174.1.

(3S, 5R)-3-Amino-5-methyl-octanoic acid hydrochloride. A slurry of (3S, 5R)-amino-5-methyl-octanoic acid, *tert*-butyl ester (2.59g, 11.3 mmol) in 6N HCl (100 mL) was heated under reflux 18 hours, cooled, and filtered over Celite. The filtrate was concentrated in vacuo to 25 mL and the resulting crystals were collected and dried to provide (3S, 5R)-3-amino-5-methyl-octanoic acid hydrochloride, mp 142.5-142.7°C (1.2g, 50.56%). A second crop (0.91g) was obtained from the filtrate. Anal. Calc'd for C₉H₁₉NO₂·HCl: C: 51.55, H: 9.61, N: 6.68, Cl: 16.91. Found: C: 51.69, H: 9.72, N: 6.56, Cl: 16.63.

(3S, 5R)-3-Amino-5-methyl-octanoic acid hydrochloride acid salt. 5.3 g of 2S-(2R-methyl-pentyl)-succinic acid-4-tert-butyl ester contained in 30 mL methyltertbutyl ether is reacted at room temperature with 3.5 mL triethylamine followed by 6.4 g of diphenylphosphoryl azide. After allowing the reaction to exotherm to 45°C and stirring for at least 4 hours, the reaction mixture is allowed to cool to room temperature and stand while the phases separated. The lower layer is discarded and the upper layer is washed with water, followed by dilute aqueous HCl. The upper layer is then combined with 10 mL of 6 N aqueous HCl, and stirred at 45-65°C. The reaction mixture is concentrated by vacuum distillation to about 10 -14 mL and allowed to crystallize while cooling to about 5°C. After collecting the product by filtration, the product is washed with toluene and reslurried in toluene. The product is dried by heating under vacuum resulting in 2.9 g (67%) of white crystalline product. The product may be recrystallized from aqueous HCl. mp 137°C.

### Example 2. (3S, 5R)-Amino-5-methyl-heptanoic acid

Methanesulfonic acid (S)-3,7-dimethyl-oct-6-enyl ester. To S-(-)-citronellol (42.8 g, 0.274 mol) and triethylamine (91 mL, 0.657 mol) in CH₂Cl₂ (800 mL) at 0°C was added methanesulphonyl chloride (26 mL, 0.329 mol) in CH₂Cl₂ (200 mL). After 2 hours at 0°C the solution was washed with 1N HCl then brine. The organic phase was dried (MgSO₄) and concentrated to afford the titled compound an oil (60.5 g, 94%) which was used without further purification. MS, *m*/*z* (relative intensity): 139 [100%], 143 [100%].

(R)-2,6-Dimethyl-oct-2-ene. To methanesulfonic acid (S)-3,7-dimethyl-oct-6-enyl ester (60 g, 0.256 mol) in THF (1 L) at 0°C was added lithium aluminum hydride (3.8 g, 0.128 mol). After 7 hours, a further 3.8 g of lithium aluminum hydride was added and the solution warmed to room temperature. After 18 hours, a further 3.8 g of lithium aluminum hydride was added. After a further 21 hours, the reaction was carefully quenched with 1N citric acid and the solution diluted further with brine. The resultant two phases were separated and the organic phase was dried (MgSO₄) and concentrated to afford the titled compound as an oil which was used without further purification. MS, *m*/*z* (relative intensity): 139 [M+H, 100%].

(R)-4-Methyl-hexanoic acid. A procedure similar to the synthesis of (R)-4-methyl-heptanoic acid was utilized giving the acid as an oil (9.3 g, 56%). MS, *m*/*z* (relative intensity): 129 [M-H, 100%].

(4R,5S)-4-Methyl-3-((R)-4-methyl-hexanoyl)-5-phenyl-oxazolidin-2-one. A procedure similar to the synthesis of (4R,5S)-4-methyl-3-((R)-4-methyl-heptanoyl)-5-phenyl-oxazolidin-2-one was utilized giving the titled compound as an oil (35.7 g, 95%). MS, *m*/*z* (relative intensity): 290 [M+H, 100.

(3S,5R)-5-Methyl-3-[1-((4R,5S)-4-methyl-2-oxo-5-phenyl-oxazolidin-3-yl)-methanoyl]-heptanoic acid tert-butyl ester. A procedure similar to the preparation of (3S,5R)-5-methyl-3-((4R,5S)-4-methyl-2-oxo-5-phenyl-oxazolidine-3-carbonyl)-octanoic acid tert-butyl ester was followed giving the titled compound as an oil (7.48 g; 31%). MS, *m*/*z* (relative intensity): 178 [100%], 169 [100%]; [α]_{D} = + 21.6 (c 1 in CHCl₃).

(S)-2-((R)-2-Methyl-butyl)-succinic acid 4-tert-butyl ester. (3S,5R)-5-Methyl-3-[1-((4R,5S)-4-methyl-2-oxo-5-phenyl-oxazolidin-3-yl)-methanoyl]-heptanoic acid tert-butyl ester (7.26 g, 0.018 mol) in H₂O (53 mL) and THF (176 mL) at 0°C was added a premixed solution of LiOH (37 mL of a 0.8 M solution) and H₂O₂ (10.57 mL of a 30% solution) and the solution warmed to room temperature. After 2 hours sodium bisulfite (7 g), sodium sulfite (13 g), and water (60 mL) was added and the two layers were separated and the aqueous layer extracted with ether. The combined organic phases were concentrated to an oil that was dissolved in heptane (200 mL). The resultant solid was filtered off and the filtrate dried (MgSO₄) and concentrated to afford the titled compound as an oil (4.4 g) that was used without further purification. MS, *m*/*z* (relative intensity): 243 [100%].

(3S, 5R)-3-Benzyoxycarbonylamino-5-methyl-heptanoic acid, *tert*-butyl ester. This compound was prepared as described above starting with (S)-2-((R)-2-methyl-butyl) succinic acid, 4-*tert*-butyl ester to give (3S, 5R)-3-benzyoxycarbonylamino-5-methyl-heptanoic acid, *tert*-butyl ester as an oil (73.3% yield). ¹H NMR (400 MHz; CDCl₃) δ 0.84(t, 3H, *J* = 7.33 Hz), 0.89(d, 3H, *J* = 6.60 Hz), 1.12-1.38 (m, 4H), 1.41 (s, 9H), 1.43-1.59 (m, 2H), 2.42 (m, 2H), 4.05 (m, 1H), 5.07 (t, 2H *J* =12.95 Hz), and 7.28-7.34 (m, 5H).

(3S, 5R)-Amino-5-methyl-heptanoic acid, *tert*-butyl ester- This compound was prepared as described above starting with (3S, 5R)-3-benzyoxycarbonylamino-5-methyl-heptanoic acid, *tert*-butyl ester instead of (3S, 5R)-3-benzyoxycarbonylamino-5-methyl-octanoic acid, *tert*-butyl ester to give the titled compound. ¹H NMR (400 MHz; CDCl₃) δ 0.84 (overlapping t and d, 6H), 1.08-1.16(m, 2H), 1.27-1.30(m, 2H), 1.42(s, 9H), 1.62 (br s, 2H), 2.15 (dd, 1H, *J*= 8.54 and 15.62 Hz), 2.29(dd, 1H, *J* = 4.15 and 15.37 Hz), and 3.20(br s, 2H).

(3S, 5R)-Amino-5-methyl-heptanoic acid hydrochloride-A slurry of (3S, 5R)-amino-5-methyl-heptanoic acid, *tert*-butyl ester (1.44g, 6.69 mmol) in 3N HCl was heated at reflux for 3 hours, filtered hot over Celite, and concentrated to dryness. Trituration of the resulting solid in ethyl ether provided (3S, 5R)-3-amino-5-methyl-heptanoic acid hydrochloride, (0.95g, 85%) mp 126.3-128.3°C.

### Example 3. (3S, 5R)-3-Amino-5-methyl-nonanoic acid

(R)-4-Methyl-octanoic acid. Lithium chloride (0.39 g, 9.12 mmol) and copper (I) chloride (0.61 g, 4.56 mmol) were combined in 45 ml THF at ambient temperature and stirred 15 minutes, then cooled to 0°C at which time ethylmagnesium bromide (1 M solution in THF, 45 mL, 45 mmol) was added. (*S*)-citronellyl bromide (5.0 g, 22.8 mmol) was added dropwise and the solution was allowed to warm slowly to ambient temperature with stirring overnight. The reaction was quenched by cautious addition of sat. NH₄Cl (aq), and stirred with Et₂O and sat. NH₄Cl (aq) for 30 minutes. The phases were separated and the organic phase dried (MgSO₄) and concentrated. The crude (R)-2,6-dimethyl-dec-2-ene was used without purification. To a solution of (R)-2,6-dimethyl-dec-2-ene (3.8 g, 22.8 mmol) in 50 mL acetone at 0°C was added Jones' reagent (2.7 M in H₂SO₄ (aq), 40 mL, 108 mmol) and the solution was allowed to warm slowly to ambient temperature with stirring overnight. The mixture was partitioned between Et₂O and H₂O, the phases were separated, and the organic phase washed with brine, dried (MgSO₄), and concentrated. The residue was purified by flash chromatography (8:1 hexanes:EtOAc) to afford 2.14 g (59%) of the titled compound as a colorless oil: LRMS: *m*/*z* 156.9 (M+). Jones' reagent was prepared as a 2.7M solution by combining 26.7g CrO₃, 23 mL H₂SO₄, and diluting to 100 mL with H₂O.

(4R, 5S)-4-Methyl-3-((R)-4-methyl-octanoyl)-5-phenyl-oxazolidin-2-one. To (R)-4-methyl-octanoic acid (2.14 g, 13.5 mmol) in 25 mL CH₂Cl₂ at 0°C was added 3 drops DMF, followed by oxalyl chloride (1.42 mL, 16.2 mmol) resulting in vigorous gas evolution. The solution was warmed directly to ambient temperature, stirred 30 minutes, and concentrated. Meanwhile, to a solution of the oxazolidinone (2.64 g, 14.9 mmol) in 40 mL THF at -78°C was added *n*-butyllithium (1.6 M soln in hexanes, 9.3 mL, 14.9 mmol) dropwise. The mixture was stirred for 10 minutes at which time the acid chloride in 10 mL THF was added dropwise. The reaction was stirred 30 minutes at -78°C, then warmed directly to ambient temperature and quenched with sat. NH₄Cl. The mixture was partitioned between Et₂O and sat. NH₄Cl (aq), the phases were separated, and the organic phase dried (MgSO₄), and concentrated to furnish 3.2 g of the titled compound as a colorless oil. LRMS: *m*/*z* 318.2 (M+).

(3S,5R)-5-Methyl-3-((4R,5S)-4-methyl-2-oxo-5-phenyl-oxazolidine-3-carbonyl)-nonanoic acid tert-butyl ester. To a solution of diisopropylamine (1.8 mL, 12.6 mmol) in 30 mL THF at -78°C was added *n*-butyllithium (1.6 M soln in hexanes, 7.6 mL, 12.1 mmol), and the mixture stirred 10 minutes at which time (4R, 5S)-4-Methyl-3-((R)-4-methyl-octanoyl)-5-phenyl-oxazolidin-2-one (3.2 g, 10.1 mmol) in 10 mL THF was added dropwise. The solution was stirred for 30 minutes, *t*-butyl bromoacetate (1.8 mL, 12.1 mmol) was added quickly dropwise at -50°C, and the mixture was allowed to warm slowly to 10°C over 3 hours. The mixture was partitioned between Et₂O and sat. NH₄Cl (aq), the phases were separated, and the organic phase dried (MgSO₄), and concentrated. The residue was purified by flash chromatography (16:1 to 8:1 hexanes:EtOAc) to provide 2.65 g (61%) of the titled compound as a colorless crystalline solid, mp = 84-86°C. [δ]_{D}²³ +17.1 (c = 1.00, CHCl₃).

(S)-2-((R)-2-Methyl-hexyl)-succinic acid 4-tert-butyl ester. To a solution of (3S,5R)-5-Methyl-3-((4R,5S)-4-methyl-2-oxo-5-phenyl-oxazolidine-3-carbonyl)-nonanoic acid tert-butyl ester (2.65 g, 6.14 mmol) in 20 mL THF at 0°C was added a precooled (0°C) solution of LiOH monohydrate (1.0 g, 23.8 mmol) and hydrogen peroxide (30 wt% aqueous soln, 5.0 mL) in 10 mL H₂O. The mixture was stirred vigorously for 90 minutes, then warmed to ambient temperature and stirred 90 minutes. The reaction was quenched at 0°C by addition of 100 mL 10% NaHSO₃ (aq), then extracted with Et₂O. The phases were separated, and the organic phase washed with brine, dried (MgSO₄), and concentrated. The titled compound was used without purification.

(3S, 5R)-3-Benzyoxycarbonylamino-5-methylnonanoic acid, *tert*-butyl ester-This compound was prepared similarly as described above starting with (S)-2-((R)-2-methylhexyl) succinic acid, 4-tert-butyl ester instead of (S)-2-((R)-2-methylpentyl) succinic acid, 4-*tert-*butyl ester to provide the titled compound as an oil (71.6% yield). ¹HNMR (400 MHz; CDCl₃) δ 0.81(t, 3H, *J* = 4.40 Hz), 0.85(d, 3H, *J* = 6.55 Hz), 1.06-1.20(m, 7H), 1.36(s, 9H), 1.38-1.50(m, 2H), 2.36(m, 2H), 3.99(m, 1H), 5.02(m+s, 3H), and 7.28-7.28(m, 5H).

(3S, 5R)-3-Amino-5-methyl-nonanoic acid, *tert*-butyl ester- This compound was prepared as described above starting with (3S, 5R)-benzyoxycarbonylamino-5-methyl-nonanoic acid, *tert*-butyl ester instead of (3S, 5R)-3-benzyoxycarbonylamino-5-methyl-octanoic acid, *tert*-butyl ester. Yield = 97%. ¹HNMR (400 MHz; CDCl₃) δ 0.82(overlapping d and t, 6H), 1.02-1.08(m, 1H), 1.09-1.36(m, 6H), 1.39(s, 9H), 1.47(br s, 1H), 1.80(s, 2H), 2.13(dd, 1H, *J* = 8.54 and 15.61 Hz), and 2.27(dd, 1H, *J* = 4.15 and 15.38 Hz).

(3S, 5R)-3-Amino-5-methyl-nonanoic acid hydrochloride-A mixture of (3S, 5R)-3-amino-5-methyl-nonanoic acid, *tert*-butyl ester (1.50g, 6.16 mmol) in 3N HCl (100 mL) was heated at reflux for 3hours, filtered hot over Celite, and concentrated to 30mL in vacuo. The resulting crystals were collected, washed with additional 3N HCl, and dried to provide the title compound, mp 142.5-143.3°C. Additional crops were obtained from the filtrate to provide 1.03g (70.4%). Anal. Calc'd for C₁₀H₂₁NO₂·HCl: C: 53.68, H: 9.91, N: 6.26, Cl: 15.85. Found: C: 53.89, H: 10.11, N: 6.13. MS: M+1: 188.1.

### Pharmaceutical Composition Examples

In the following Examples, the term 'active compound' or 'active ingredient' refers to a suitable combination or individual element of an alpha-2-delta ligand and DSNRI or ono or SNRI and/or a pharmaceutically acceptable salt, according to the present invention.

### (i) Tablet compositions

The following compositions A and B can be prepared by wet granulation of ingredients (a) to (c) and (a) to (d) with a solution of povidone, followed by addition of the magnesium stearate and compression.

| Composition A | | |
|---|---|---|
| | mg/tablet | mg/tablet |
| (a) Active ingredient | 250 | 250 |
| (b) Lactose B.P. | 210 | 26 |
| (c) Sodium Starch Glycollate | 20 | 12 |
| (d) Povidone B.P. | 15 | 9 |
| (e) Magnesium Stearate | 5 | 3 |
| | 500 | 300 |

| Composition B | | |
|---|---|---|
| | mg/tablet | mg/tablet |
| (a) Active ingredient | 250 | 250 |
| (b) Lactose 150 | 150 | - |
| (c) Avicel PH 101 | 60 | 26 |
| (d) Sodium Starch Glycollate | 20 | 12 |
| (e) Povidone B.P. | 15 | 9 |
| (f) Magnesium Stearate | 5 | 3 |
| | 500 | 300 |

| Composition C | |
|---|---|
| | mg/tablet |
| Active ingredient | 100 |
| Lactose | 200 |
| Starch | 50 |
| Povidone | 5 |
| Magnesium Stearate | 4 |
| | 359 |

The following compositions D and E can be prepared by direct compression of the admixed ingredients. The lactose used in formulation E is of the direct compression type.

| Composition D | |
|---|---|
| | mg/tablet |
| Active ingredient | 250 |
| Magnesium Stearate | 4 |
| Pregelatinised Starch NF15 | 146 |
| | 400 |

| Composition E | |
|---|---|
| | mg/tablet |
| Active ingredient | 250 |
| Magnesium Stearate | 5 |
| Lactose | 145 |
| Avicel | 100 |
| | 500 |

| Composition F (Controlled release composition) | | |
|---|---|---|
| | | mg/tablet |
| (a) | Active ingredient | 500 |
| (b) | Hydroxypropylmethylcellulose (Methocel K4M Premium) | 112 |
| (c) | Lactose B.P. | 53 |
| (d) | Povidone B.P.C. | 28 |
| (e) | Magnesium Stearate | 7 |
| | | 700 |

The composition can be prepared by wet granulation of ingredients (a) to (c) with a solution of povidone, followed by addition of the magnesium stearate and compression.

### Composition G (Enteric-coated tablet)

Enteric-coated tablets of Composition C can be prepared by coating the tablets with 25mg/tablet of an enteric polymer such as cellulose acetate phthalate, polyvinylacetate phthalate, hydroxypropylmethyl-cellulose phthalate, or anionic polymers of methacrylic acid and methacrylic acid methyl ester (Eudragit L). Except for Eudragit L, these polymers should also include 10% (by weight of the quantity of polymer used) of a plasticizer to prevent membrane cracking during application or on storage. Suitable plasticizers include diethyl phthalate, tributyl citrate and triacetin.

### Composition H (Enteric-coated controlled release tablet)

Enteric-coated tablets of Composition F can be prepared by coating the tablets with 50mg/tablet of an enteric polymer such as cellulose acetate phthalate, polyvinylacetate phthalate, hydroxypropylmethyl- cellulose phthalate, or anionic polymers of methacrylic acid and methacrylic acid methyl ester (Eudgragit L). Except for Eudgragit L, these polymers should also include 10% (by weight of the quantity of polymer used) of a plasticizer to prevent membrane cracking during application or on storage. Suitable plasticizers include diethyl phthalate, tributyl citrate and triacetin.

### (ii) Capsule compositions

### Composition A

Capsules can be prepared by admixing the ingredients of Composition D above and filling two-part hard gelatin capsules with the resulting mixture. Composition B (infra) may be prepared in a similar manner.

| Composition B | | |
|---|---|---|
| | | mg/capsule |
| (a) | Active ingredient | 250 |
| (b) | Lactose B.P. | 143 |
| (c) | Sodium Starch Glycollate | 25 |
| (d) | Magnesium Stearate | 2 |
| | | 420 |

| Composition C | | |
|---|---|---|
| | | mg/capsule |
| (a) | Active ingredient | 250 |
| (b) | Macrogol 4000 BP | 350 |
| | | 600 |

Capsules can be prepared by melting the Macrogol 4000 BP, dispersing the active ingredient in the melt and filling two-part hard gelatin capsules therewith.

| Composition D | |
|---|---|
| | mg/capsule |
| Active ingredient | 250 |
| Lecithin | 100 |
| Arachis Oil | 100 |
| | 450 |

Capsules can be prepared by dispersing the active ingredient in the lecithin and arachis oil and filling soft, elastic gelatin capsules with the dispersion.

| Composition E (Controlled release capsule) | | |
|---|---|---|
| | | mg/capsule |
| (a) | Active ingredient | 250 |
| (b) | Microcrystalline Cellulose | 125 |
| (c) | Lactose BP | 125 |
| (d) | Ethyl Cellulose | 13 |
| | | 513 |

The controlled release capsule formulation can be prepared by extruding mixed ingredients (a) to (c) using an extruder, then spheronising and drying the extrudate. The dried pellets are coated with a release controlling membrane (d) and filled into two-part, hard gelatin capsules.

| Composition F (Enteric capsule) | | |
|---|---|---|
| | | mg/capsule |
| (a) | Active ingredient | 250 |
| (b) | Microcrystalline Cellulose | 125 |
| (c) | Lactose BP | 125 |
| (d) | Cellulose Acetate Phthalate | 50 |
| (e) | Diethyl Phthalat | 5 |
| | | 555 |

The enteric capsule composition can be prepared by extruding mixed ingredients (a) to (c) using an extruder, then spheronising and drying the extrudate. The dried pellets are coated with an enteric membrane (d) containing a plasticizer (e) and filled into two-part, hard gelatin capsules.

### Composition G (Enteric-coated controlled release capsule)

Enteric capsules of Composition E can be prepared by coating the controlled-release pellets with 50mg/capsule of an enteric polymer such as cellulose acetate phthalate, polyvinylacetate phthalate, hydroxypropylmethylcellulose phthalate, or anionic polymers of methacrylic acid and methacrylic acid methyl ester (Eudragit L). Except for Eudragit L, these polymers should also include 10% (by weight of the quantity of polymer used) or a plasticizer to prevent membrane cracking during application or on storage. Suitable plasticizers include diethyl phthalate, tributyl citrate and triacetin.

### (iii) Intravenous injection composition

| | |
|---|---|
| Active ingredient | 0.200g |
| Sterile, pyrogen-free phosphate buffer (pH 9.0) to | 10 ml |

The active ingredient is dissolved in most of the phosphate buffer at 35-40°C, then made up to volume and filtered through a sterile micropore filter into sterile 10 ml glass vials (Type 1) which are sealed with sterile closures and overseals.

### (iv) Intramuscular injection composition

| | |
|---|---|
| Active ingredient | 0.20 g |
| Benzyl Alcohol | 0.10 g |
| Glycofurol 75 | 1.45 g |
| Water for Injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml glass vials (Type 1).

### (v) Syrup composition

| | |
|---|---|
| Active ingredient | 0.25g |
| Sorbitol Solution | 1.50g |
| Glycerol | 1.00g |
| Sodium Benzoate | 0.005g |
| Flavour | 0.0125ml |
| Purified Water q.s. to | 5.0ml |

The sodium benzoate is dissolved in a portion of the purified water and the sorbitol solution added. The active ingredient is added and dissolved. The resulting solution is mixed with the glycerol and then made up to the required volume with the purified water.

### (vi) Suppository composition

| | mg/suppository |
|---|---|
| Active ingredient | 250 |
| Hard Fat, BP (Witepsol H15 - Dynamit NoBel) | 1770 |
| | 2020 |

One-fifth of the Witepsol H15 is melted in a steam-jacketed pan at 45°C maximum. The active ingredient is sifted through a 200lm sieve and added to the molten base with mixing, using a Silverson fitted with a cutting head, until a smooth dispersion is achieved.

Maintaining the mixture at 45°C, the remaining Witepsol H15 is added to the suspension which is stirred to ensure a homogenous mix. The entire suspension is then passed through a 250lm stainless steel screen and, with continuous stirring, allowed to cool to 40°C. At a temperature of 38-40°C, 2.02g aliquots of the mixture are filled into suitable plastic moulds and the suppositories allowed to cool to room temperature.

### (vii) Pessary composition

| | mg/pessary |
|---|---|
| Active ingredient (63lm) | 250 |
| Anhydrous Dextrose | 380 |
| Potato Starch | 363 |
| Magnesium Stearate | 7 |
| | 1000 |

The above ingredients are mixed directly and pessaries prepared by compression of the resulting mixture.

### (viii) Transdermal composition

| | |
|---|---|
| Active ingredient | 200mg |
| Alcohol USP | 0.1ml |
| Hydroxyethyl cellulose | |

The active ingredient and alcohol USP are gelled with hydroxyethyl cellulose and packed in a transdermal device with a surface area of 10cm².

## Claims

1. A combination consisting essentially of a synergistic amount of an alpha-2-delta ligand, wherein the alpha-2-delta ligand is selected from the group consisting of gabapentin and pregabalin, and (S, S)-reboxetine, or a pharmaceutically acceptable salt of any thereof.

2. A combination according to Claim 1, for the curative, prophylactic or palliative treatment of pain.

3. A combination according to Claim 2, wherein the pain is neuropathic pain.

4. A pharmaceutical composition for the curative, prophylactic or palliative treatment of pain comprising a therapeutically effective amount of a combination according to Claim 1 and a suitable carrier or excipient.

5. Use of a synergistic effective amount of an alpha-2-delta ligand, wherein the alpha-2-delta ligand is selected from the group consisting of gabapentin and pregabalin, and (S, S)-reboxetine, or a pharmaceutically acceptable salt of any thereof, in the manufacture of a medicament for the cuarative, prophylactic or palliative treatment of pain.

6. Use according to Claim 5 where the pain is neuropathic pain.

7. A kit comprising:
a. an alpha-2-delta ligand selected from the group consisting of gabapentin and pregabalin, or a pharmaceutically acceptable salt thereof;
b. (S, S)-reboxetine or a pharmaceutically acceptable salt thereof; and c. a container.

## Patentansprüche

1. Kombination, im Wesentlichen bestehend aus einer synergistischen Menge eines Alpha-2-delta-Liganden, wobei der Alpha-2-delta-Ligand aus der Gruppe, bestehend aus Gabapentin und Pregabalin, ausgewählt ist, und (S,S)-Reboxetin oder eines pharmazeutisch verträglichen Salzes von jedem davon.

2. Kombination gemäß Anspruch 1 für die heilende, prophylaktische oder palliative Behandlung von Schmerz.

3. Kombination gemäß Anspruch 2, wobei der Schmerz neuropathischer Schmerz ist.

4. Pharmazeutische Zusammensetzung für die heilende, prophylaktische oder palliative Behandlung von Schmerz, umfassend eine therapeutisch wirksame Menge einer Kombination gemäß Anspruch 1 und einen geeigneten Träger oder ein geeignetes Exzipiens.

5. Verwendung einer synergistischen wirksamen Menge eines Alpha-2-delta-Liganden, wobei der Alpha-2-delta-Ligand aus der Gruppe, bestehend aus Gabapentin und Pregabalin, ausgewählt ist, und (S,S)-Reboxetin oder eines pharmazeutisch verträglichen Salzes von jedem davon, bei der Herstellung eines Medikaments für die heilende, prophylaktische oder palliative Behandlung von Schmerz.

6. Verwendung gemäß Anspruch 5, wobei der Schmerz neuropathischer Schmerz ist.

7. Kit, umfassend:
a. einen Alpha-2-delta-Liganden, ausgewählt aus der Gruppe, bestehend aus Gabapentin und Pregabalin, oder ein pharmazeutisch verträgliches Salz davon;
b. (S,S)-Reboxetin oder ein pharmazeutisch verträgliches Salz davon und
c. einen Behälter.

## Revendications

1. Combinaison consistant essentiellement en une quantité synergique d'un ligand alpha-2-delta, le ligand alpha-2-delta étant sélectionné dans le groupe consistant en la gabapentine, la prégabaline et la (*S,S*)-réboxétine, ou en un sel pharmaceutiquement acceptable de l'un quelconque de ces composés.

2. Combinaison selon la revendication 1, pour le traitement curatif, prophylactique ou palliatif de la douleur.

3. Combinaison selon la revendication 2, dans laquelle la douleur est la douleur neuropathique.

4. Composition pharmaceutique pour le traitement curatif, prophylactique ou palliatif de la douleur, comprenant une quantité thérapeutiquement efficace d'une combinaison selon la revendication 1 et un support ou excipient convenable.

5. Utilisation d'une quantité efficace, synergique, d'un ligand alpha-2-delta, le ligand alpha-2-delta étant sélectionné dans le groupe consistant en la gabapentine, la prégabaline et la (*S,S*)-réboxétine, ou en un sel pharmaceutiquement acceptable de l'un quelconque de ces composés, dans la fabrication d'un médicament pour le traitement curatif, prophylactique ou palliatif de la douleur.

6. Utilisation selon la revendication 5, dans laquelle la douleur est la douleur neuropathique.

7. Kit comprenant :
a. un ligand alpha-2-delta sélectionné dans le groupe consistant en la gabapentine et la prégabaline, ou en un sel pharmaceutiquement acceptable correspondant ;
b. d e la (*S,S*)-réboxétine ou un sel pharmaceutiquement acceptable correspondant ; et
c. un récipient.
